# EUROPEAN PATENT SPECIFICATION

(11) **EP 1 247 796 B1**
(45) Date of publication and mention of the grant of the patent: **13.05.2009**
(21) Application number: 02005204.9
(22) Date of filing: 07.03.2002
(51) Int. Cl.: C07C 69/92, C09K 19/32, C07C 49/755, C07C 45/74

(54) **Photoisomerizable compounds**
Photopolymerisierbare Verbindungen
Composés photopolymérisables

(30) Priority: 06.04.2001 EP 01108720
(43) Date of publication of application: 09.10.2002
(73) Proprietor: Merck Patent GmbH, 64293 Darmstadt (DE)
(72) Inventor: Farrand, Louise Diane, Davyhulme, Manchester M41 7NT (GB); Worrall, Christopher, Northwich, Cheshire CW9 7SR (GB); Parri, Owain Llyr, Dorset BH15 2LN (GB)

(56) References cited:
- WO-A-99/04789
- YAMAGUCHI T. RT AL.: "Photochromism of hemithioindigo derivatives. I. Preparation and photochromic properties in organic solvents" BULLETIN OF THE CHEMICAL SOCIETY OF JAPAN., vol. 65, no. 3, 1992, pages 649-656, XP002250487 JAPAN PUBLICATIONS TRADING CO. TOKYO., JP ISSN: 0009-2673
- SEIKI T. ET AL.: "Photochromism of hemithioindigo derivatives. II. Photochromic behaviors in bilayer membranes and related systems" BULLETIN OF THE CHEMICAL SOCIETY OF JAPAN., vol. 65, no. 3, 1992, pages 657-663, XP002250488 JAPAN PUBLICATIONS TRADING CO. TOKYO., JP ISSN: 0009-2673
- CHEMICAL ABSTRACTS, vol. 118, no. 6, 8 February 1993 (1993-02-08) Columbus, Ohio, US; abstract no. 48951, KAJI, MAKOTO: "Nonlinear optical material containing benzalindanone compound" XP002250489 & JP 04 233520 A (HITACHI CHEMICAL CO., LTD., JAPAN) 21 August 1992 (1992-08-21)

## Description

The invention relates to novel photoisomerizable compounds, to liquid crystalline mixtures and polymers containing them, and to the use of the novel compounds, mixtures and polymers in optical and electrooptical devices like liquid crystal displays or projection systems, patterned films and optical elements like polarizers, retardation films, compensators, colour filters, holographic elements or polarization beam splitters, as sensitizers, for photoswitching, in anisotropic membranes for the permeation of gases or fluids, adhesives, synthetic resins with anisotropic mechanical properties, cosmetic or pharmaceutical compositions, UV absorbers and sunscreens, diagnostics or liquid crystal pigments, for decorative and security applications, in nonlinear optics, optical information storage or as dopants.

### Background and Prior Art

In prio art photoisomerizable mesogenic molecules are reported which change their shape on photo-irradiation. When irradiated e.g. with UV light, they show E-Z or cis-trans isomerization.

Photoisomerizable compounds can be used in liquid crystal host mixtures as switchable materials or for changing the birefringence of the host mixture. Polymerizable liquid crystal mixtures comprising photoisomerizable compounds can be used for the preparation of patterned anisotropic polymer films. The photoisomerizable compounds and the liquid crystal mixtures and polymer films prepared thereof can be used for example as optical element like colour filters or polarization beam splitters, in information storage device, anisotropic membranes for the permeation of gases, or in photoswitching devices.

### Compounds of the formula

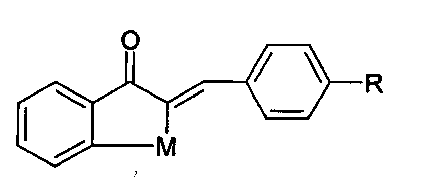

wherein R is an organic residue and M is short alkylene or heteroalkylene, undergo *EZ* photoisomerisation when irradiated with UV light, thus changing from the *E* conformation to the *Z* conformation as shown below.

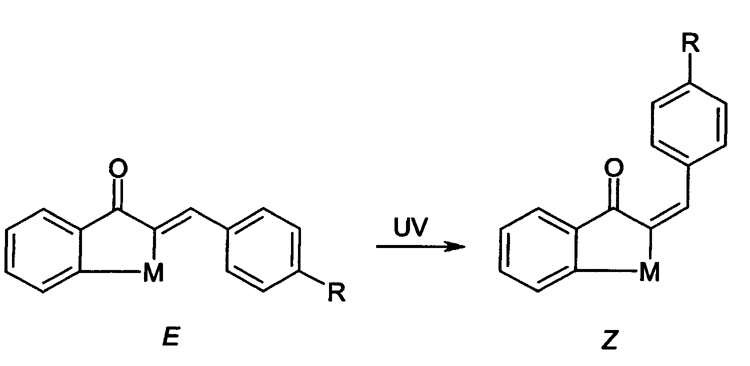

The inventors found that when these compounds are added to a liquid crystal host mixture, the *EZ* photoisomerisation disrupts the parallel stacking of the mesogens in the liquid crystal mixture, leading to a change of the birefringence. If so desired, the above compounds can even be used to cause a transition from the liquid crystal phase to the isotropic phase.

Furthermore the inventors found that compounds of the above formula wherein R is a mesogenic group and/or a polymerizable group are particular useful for the preparation of liquid crystal mixtures for photoswitching or the preparation of patterned polymer films. Compounds of the above formula that show mesogenic phase behaviour are particularly useful, as they do not negatively affect the liquid crystalline phase of the host mixture.

JP 04-233520 A discloses a nonlinear optical material comprising indanone derivatives of the following formula 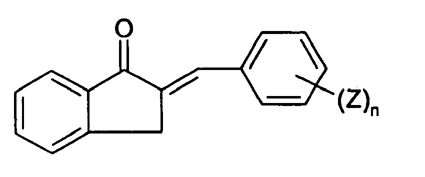 wherein n is 1 to 5 and Z is C₁₋₁₀-alkyl, C₁₋₁₀-alkoxy, aryl, aralkyl, aralkyloxy, haloalkyl, Br or Cl. The indanone derivatives are said to generate a large dipole moment by charge transfer in an excited state to give high super polarizibility.

US 5,112,721, US 4,162,162 and EP 0 483 648 disclose indanone derivatives of the formula 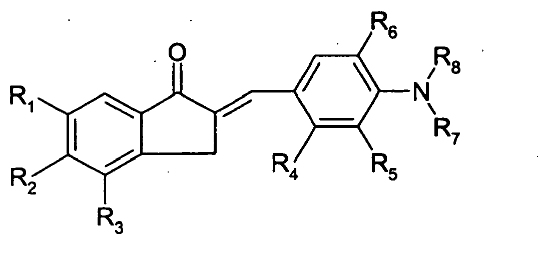 wherein R₁ to R₈ are hydrogen or alkyl, for use as coinitiators sensitizers in photopolymerizable compositions.

Yamaguchi et al., Bull. Chem. Soc. Jpn. 65, 649-656 (1992) and Seki et al., Bull. Chem. Soc. Jpn. 65, 657-663 (1992), disclose hemithioindigo compounds showing E/Z-isomerisation. WO 99/04789 A1 and JP 04-233520 A show indanone compounds.

However, the above prior art documents do not give a hint to mesogenic or polymerizable compounds according to the present invention, or to their use as photoisomerizable compounds for the preparation of patterned polymer films.

The invention has the aim of providing new compounds, in particular polymerizable and/or mesogenic compounds, which are suitable as photoisomerizable compounds and for other uses, are easy to synthesize in a large range of derivatives, preferably show broad liquid crystalline phases with high clearing points and, when added to a liquid crystalline host mixture, do not greatly affect the properties of the host mixture. Another aim of the invention is to provide liquid crystal mixtures comprising photoisomerizable compounds.

It has been found that the above aims can be achieved by providing mixtures and compounds as claimed in the present invention.

### Definition of Terms

The terms 'liquid crystalline or mesogenic material' or 'liquid crystalline or mesogenic compound' should denote materials or compounds comprising one or more rod-shaped, lath-shaped or disk-shaped mesogenic groups, i.e. groups with the ability to induce liquid crystal phase behaviour. Rod-shaped and lath-shaped mesogenic groups are especially preferred. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerized.

The term 'reactive mesogen' means a polymerizable mesogenic compound.

For the sake of simplicity, the term 'liquid crystal material' is used hereinafter for both liquid crystal materials and mesogenic materials.

The term 'film' includes self-supporting, i.e. free-standing, films that show more or less pronounced mechanical stability and flexibility, as well as coatings or layers on a supporting substrate or between two substrates.

The term 'photoisomerizable group' means a group that shows isomerization, for example cis-trans or E-Z isomerization, imparting a change in shape upon photoirradiation with a suitable wavelength, preferably in the range from 250 to 400 nm, very preferably from 300 to 400 nm.

### Summary of the Invention

One object of the invention is a polymerizable liquid crystalline mixture comprising at least one compound of formula I, and comprising at least one polymerizable compound, which can be a compound of formula I or an additional polymerizable compound 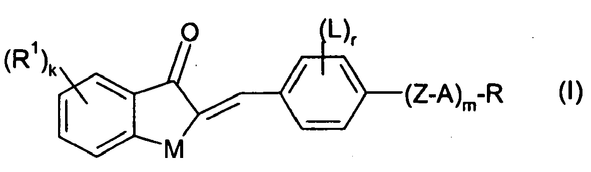 wherein
- M: is (CH₂)ᵢ, wherein one CH₂ group may also be replaced by NR⁰, O or S,
- i: is 1, 2, 3 or 4,
- R⁰: is H or alkyl with 1 to 4 C atoms,
- L: is halogen, CN, SCN, NO₂, SF₅ or an alkyl, alkoxy, alkylcarbonyl or alkoxycarbonyl group with 1 to 4 C atoms, wherein one or more H atoms may be substituted with F or Cl,
- r: is 0, 1, 2, 3 or 4,
- R¹: is in each case independently OH, O-B, OCH₂B or has one of the meanings of R given below,
- k: is 0, 1, 2, 3 or 4,
- A and B: are independently of each other an aromatic or alicyclic group with 4 to 12 C-atoms,
- Z: is in each case independently -O-, -S-, -CO-, -COO-, -OCO-, -CO-NR⁰-, -NR⁰-CO-, -CH₂CH₂-, -CF₂CF₂-, -CH₂CF₂-, -OCH₂-, -CH₂O-, -SCH₂-, -CH₂S-, -CF₂O-, -OCF₂-, -CH=CH-, -CF=CF-, -CH=CF-, -(CH₂)₄-, -CH=CH-COO-, -OCO-CH=CH-, -OOC-CHR⁰⁰-OOC-, -COO-CHR⁰⁰-COO-, -C≡C- or a single bond,
- R⁰⁰: is straight chain or branched alkyl or alkoxy with 1 to 8 C atoms or phenyl that is optionally mono- or polysubstituted by L,
- m: is 0, 1 or 2,
- R: is halogen, NO₂, CN, SCN, SF₅, straight chain, branched or cyclic alkyl with 1 to 25 C atoms wherein one or more CH₂ groups can also be replaced by -O-, -S-, -CO-, -NR⁰-, -CH=CH-, -C≡C- in such a manner that O and/or S atoms are not linked directly to one another, and wherein one or more H-atoms can also be replaced by F, Cl or phenyl that is optionally mono- or polysubstituted by L, or denotes P-(Sp-X)ₙ-,
- P: is a polymerizable group. The polymerisable group P is from CH₂=CW¹-COO-, 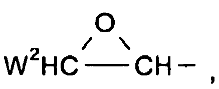 CH₂CW²-O-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, OCN- and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.
- Sp: is a spacer group with 1 to 20 C atoms,
- X: has one of the meanings of Z, and
- n: is 0 or 1, with the proviso that m is 1 or 2 and/or at least one of R and R¹ denotes P-(Sp-x)ₙ-.

Another object of the invention is a liquid crystalline mixture comprising one or more compounds of formula I.

Another object of the present invention is a polymerizable liquid crystalline mixture comprising at least two compounds, at least one of which is a compound of formula I and at least one of which is a polymerizable compound.

Another object of the invention is a compound of formula I, wherein U, R, R¹, A, Z, L, k, m and r are as defined above and below, and wherein at least one of R and R¹ denotes P-(sp-x)ₙ-.

Another object of the invention is a linear or crosslinked anisotropic polymer obtainable by polymerizing a polymerizable liquid crystalline mixture comprising one or more compounds of formula I.

Another object of the invention is the use of a compound of formula I, mixture or polymer as described above in optical and electrooptical devices like liquid crystal displays or projection systems, such as STN, TN, AMD-TN, temperature compensation, ferroelectric, guest-host, phase change or surface stabilized or polymer stabilized cholesteric texture (SSCT, PSCT) displays, in optical elements, like reflective polarizers, retardation films, compensators, colour filters, polarization beam splitters or holographic elements, especially in films with patterned optical properties, in adhesives, synthetic resins with anisotropic mechanical properties like for example anisotropic membranes for the permeation of gases or fluids, cosmetic and pharmaceutical compositions like for example UV absorbers, UV filters or sunscreens, as sensitizers, in diagnostics, liquid crystal pigments, for decorative and security applications, especially in security markings that are applied to items or documents of value for easy identification or prevention of falsification, in nonlinear optics, optical recording or information storage, or as dopants.

Another object of the invention is an anisotropic polymer film comprising a compound of formula I.

Another object of the invention is an anisotropic polymer film with patterned optical properties comprising a compound of formula I.

### Detailed Description of the Invention

Inventive compounds are preferred that are mesogenic or liquid crystalline, i.e. they can induce or enhance mesophase behaviour for example in a mixture with other compounds or exhibit one or more mesophases themselves. It is also possible that the inventive compounds show mesophase behaviour only in mixtures with other compounds, or, in case of polymerizable compounds, when being (co)polymerized. Mesogenic inventive compounds are especially preferred.

The inventive compounds have several advantages
- they can easily be synthesized, also on large scale of several hundred grams, with a broad range of derivatives using standard methods that are known from the literature,
- the starting materials can be obtained commercially or synthesized cheaply using methods known from the literature
- they exhibit a good solubility in liquid crystalline mixtures,
- they exhibit mesophase behaviour and in the ideal case broad liquid crystalline phases.

Photolysis of the compounds of formula I with e.g. UV light of 360 nm has the effect of isomerising the double bond of the cycloalkanone group from *E* to *Z*, thus completely changing the shape of the molecule and hence the physical molecular properties. The inventive compounds of formula I can be mixed with other polymerizable mesogenic compounds to give a liquid crystalline mixture. If such a liquid crystalline mixture, for example a nematic mixture is aligned on a surface and then photolysed with e.g. UV light of 360 nm, the light changes the shape of the isomerizable dopant - this in turn disrupts the parallel alignment of the mixture, and this has an overall effect of reducing the birefringence of the mixture.

If irradiation is carried out through a mask towards a liquid crystalline mixture doped with the photoisomerizable compound, and the resulting film is viewed between crossed polarisers, the change of birefringence shows a lower retardation in areas of higher UV transmission.

The compounds of formula I are thus useful e.g. for forming optical films with novel architectures.

Particularly preferred are compounds of formula I wherein M is CH₂, NR⁰, O, S, (CH₂)₂, NR⁰-CH₂, O-CH₂ or S-CH₂, very preferably CH₂ or (CH₂)₂. R° is preferably H or CH₃.

Further preferred are compounds of formula I wherein
- A: is in each case independently 1,4-phenylene in which, in addition, one or more CH groups may be replaced by N, 1,4-cyclohexylene in which, in addition, one or two non-adjacent CH₂ groups may be replaced by O and/or S, 1,3-dioxolane-4,5-diyl, 1,4-cyclohexenylene, piperidine-1,4-diyl, pyrrolidine-2,4-diyl, 1,4-bicyclo-(2,2,2)-octylene, naphthalene-2,6-diyl, decahydro-naphthalene-2,6-diyl, 1,2,3,4-tetrahydronaphthalene-2,6-diyl or indane-2,5-diyl, it being possible for all these groups to be unsubstituted, mono- or polysubstituted with L as defined in formula I.

Further preferred are compounds of formula I wherein
- one or more of R and R¹ denote P-(Sp-X)ₙ,
- R and/or R¹ are straight chain or branched, very preferably straight chain alkyl or alkoxy with 1 to 12 C atoms,
- m is 1 or 2, very preferably 1,
- k is 0 or 1,
- k is 1, 2, 3 or 4 and at least one R¹ is different from H,
- n is 1,
- M is CH₂ or OCH₂ and at least one of R and R¹ is P-(Sp-X)ₙ,
- M is CH₂ or OCH₂ and m is 1, preferably with Z not being O or a single bond,
- M is CH₂ or OCH₂ and k is 1, 2, 3 or 4.

Further preferred are compounds of formula I comprising one or two, very preferably two polymerizable groups.

The group B in formula I is preferably a monocyclic or bicyclic aromatic or alicyclic group having preferably 6 to 10 C atoms. Especially preferably B has one of the preferred meanings of A given above.

Particularly preferred are compounds of formula I wherein the group (Z-A)ₘ is selected from the group of formulae listed below. For reasons of simplicity, Phe in these formulae is 1,4-phenylene that may also be substituted with 1 to 4 groups L as defined in formula II, Cyc is 1,4-cyclohexylene and Z has one of the meanings of formula II. The list is comprising the following subformulae as well as their mirror images

-Phe- II-1

-Cyc- II-2

-Phe-Z-Phe- II-3

-Phe-Z-Cyc- II-4

-Cyc-Z-Cyc- II-5

Z is preferably -COO-, -OCO-, -CH₂CH₂- or a single bond.

A particularly preferred embodiment relates to compounds wherein at least one group Z is a chiral group OOC-CHR⁰⁰-OOC or COO-CHR⁰⁰-COO. R⁰⁰ in these groups is preferably phenyl or straight-chain or branched alkyl with 2 to 5 C atoms, very preferably phenyl or 2-methylpropyl.

Very preferably the group

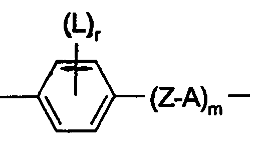

in formula I is selected from the following formulae 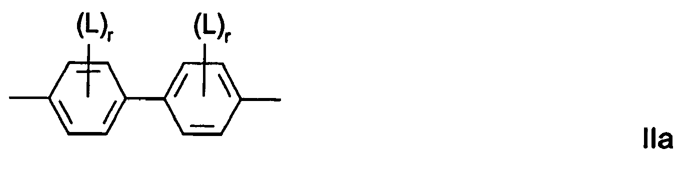 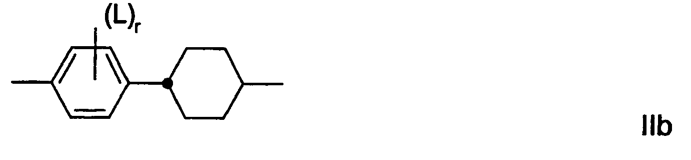 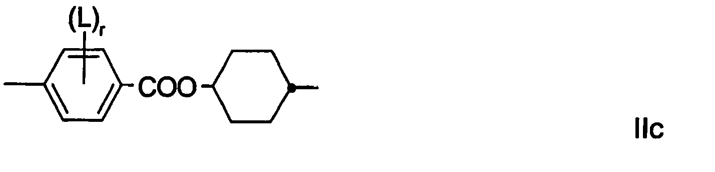 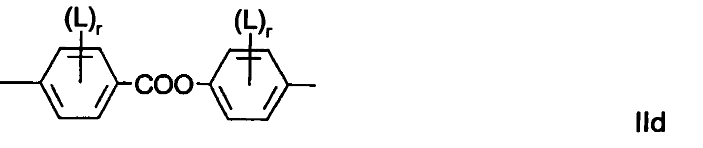 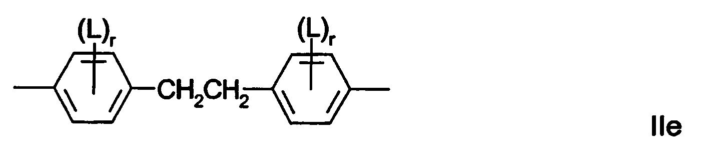 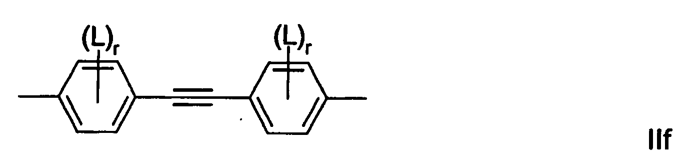 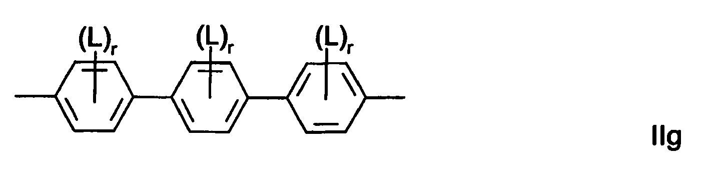 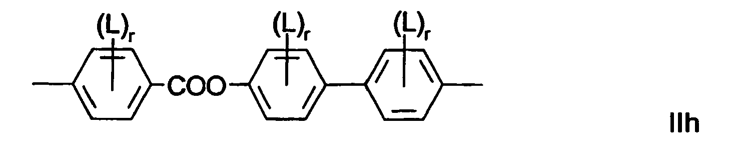 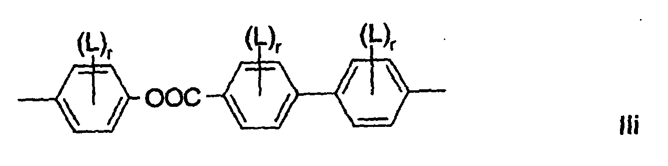 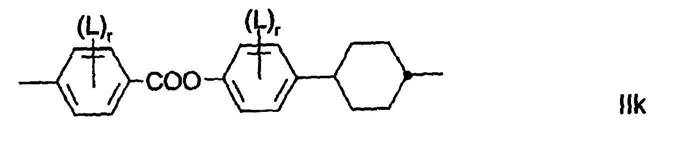 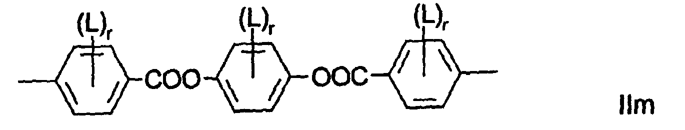 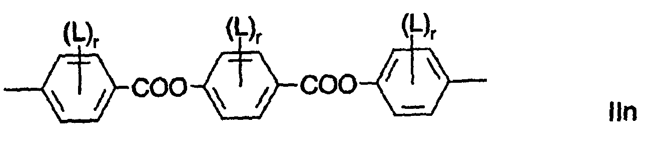 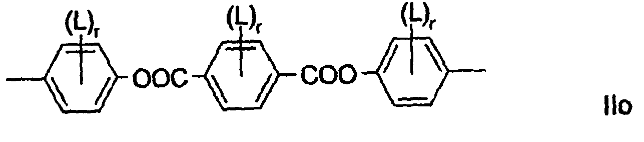 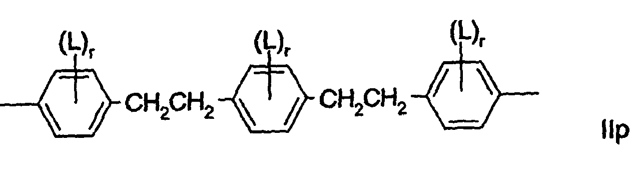 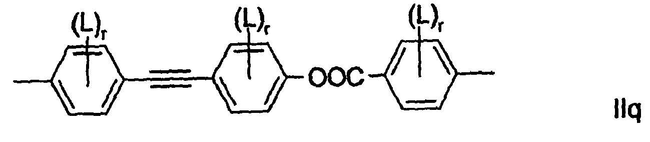 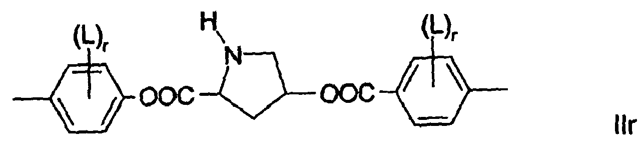 wherein L and r have the above meanings, and r is preferably 0, 1 or 2.

The group 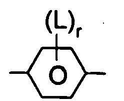 in these preferred formulae is very preferably denoting 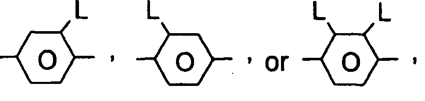 furthermore 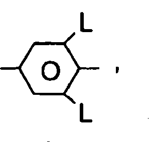 with L having each independently one of the meanings given above.

Particularly preferred are the subformulae IId, IIg, IIh, IIi, IIm, IIn and IIo, in particular the subformula lld.

L is preferably F, Cl, CN, NO₂, CH₃, C₂H₅, OCH₃, OC₂H₅, COCH₃, COC₂H₅, COOCH₃, COOC₂H₅, CF₃, OCF₃, OCHF₂ or OC₂F₅, in particular F, Cl, CN, CH₃, C₂H₅, OCH₃, COCH₃, CF₃ or OCF₃ ,most preferably F, Cl, CH₃, OCH₃ or OCF₃.

Particularly preferred compounds of formula I are those of the following subformulae 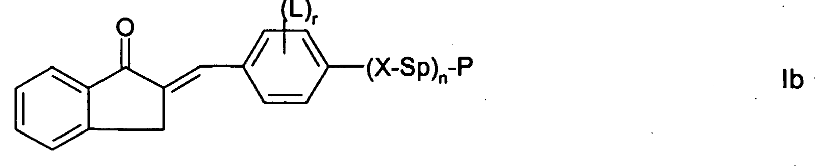 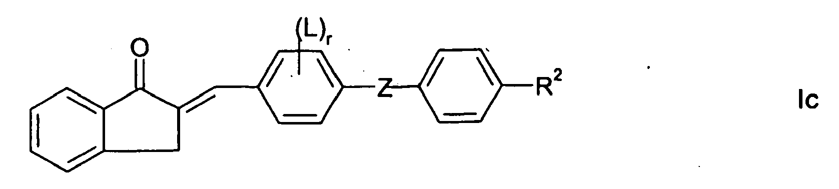 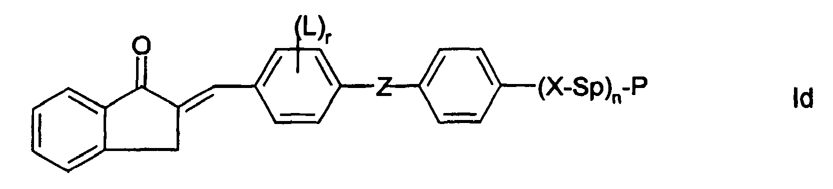 wherein Z, P, Sp, X, L, n and r are as defined in formula I and
- R²: is H, halogen, NO₂, CN, SCN, SF₅, straight chain, branched or cyclic alkyl with 1 to 25 C atoms wherein one or more CH₂ groups can also be replaced by -O-, -S-, -CO-, -NR⁰-, -CH=CH-, -C≡C- in such a manner that O and/or S atoms are not linked directly to one another, and wherein one or more H-atoms can also be replaced by F or Cl.

Particularly preferred are compounds of formula la to Id wherein r is 0. Further preferred are compounds of formula Ib and Id wherein Z is COO, OCO, CH₂CH₂ or a single bond.

If R, R¹ or R² is an alkyl or alkoxy radical, i.e. where the terminal CH₂ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Especially preferred is straight chain alkyl or alkoxy with 1 to 8 C atoms.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

R, R¹ and R² can be a polar or an unpolar group.

In case of a polar group, it is preferably selected from CN, NO₂, halogen, OCH₃, SCN, COR⁸, COOR⁸ or a mono- oligo- or polyfluorinated alkyl or alkoxy group with 1 to 4 C atoms. R⁸ is optionally fluorinated alkyl with 1 to 4, preferably 1 to 3 C atoms. Especially preferably polar groups are selected of F, Cl, CN, NO₂, OCH₃, COCH₃, COC₂H₅, COOCH₃, COOC₂H₅, CF₃, C₂F₅, OCF₃, OCHF₂, and OC₂F₅, in particular of F, Cl, CN, OCH₃ and OCF₃.

In case of an unpolar group, it is preferably alkyl with up to 15 C atoms or alkoxy with 2 to 15 C atoms.

R, R¹ and R² can be an achiral or a chiral group. In case of a chiral group, it is preferably selected according to formula IV: 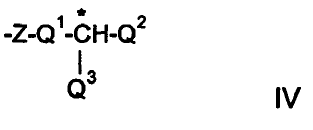 wherein
- Z³: is -O-, -S-, -CO-, -COO-, -OCO-, -OCOO- or a single bond,
- Q¹: is an alkylene or alkylene-oxy group with 1 to 9 C atoms or a single bond,
- Q²: is an alkyl or alkoxy group with 1 to 10 C atoms which may be unsubstituted, mono- or polysubstituted with halogen or CN, it being also possible for one or more non-adjacent CH₂ groups to be replaced, in each case independently from one another, by -C≡C-, -O-, -S-, -NR⁰-, -CO-, -COO-, -OCO-, -OCO-O-, -S-CO- or -CO-S- in such a manner that oxygen atoms are not linked directly to one another,
- Q³: is halogen, a cyano group, an alkyl or alkoxy group with 1 to 4 C atoms different from Q², or a phenyl group that may also be mono- or polysubstituted with L as defined in formula I.

In case Q¹ in formula IV is an alkylene-oxy group, the O atom is preferably adjacent to the chiral C atom.

Preferred chiral groups are 2-alkyl, 2-alkoxy, 2-carbonyl, 2-carbonyloxy, 2-oxycarbonyl, 2-methylalkyl, 2-methylalkoxy, 2-methylcarbonyl, 2-methylcarbonyloxy, 2-methyloxycarbonyl, 2-fluoroalkyl, 2-fluoroalkoxy, 2-(2-ethin)-alkyl, 2-(2-ethin)-alkoxy, 1,1,1-trifluoro-2-alkyl, 1,1,1-trifluoro-2-alkoxy, 2-phenylalkyl, 2-phenylalkoxy, 2-phenylcarbonyl, 2-phenylcarbonyloxy and 2-phenyloxycarbonyl.

Particularly preferred chiral groups are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy, 2-ethylhexoxy, 1-methylhexoxy, 2-octyloxy, 2-oxa-3-methylbutyl, 3-oxa-4-methylpentyl, 4-methylhexyl, 2-hexyl, 2-octyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy, 2-phenylbutyl, 2-phenylbutyloxy, 2-phenylpropylcarbonyl, 2-phenylpropylcarbonyloxy, for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy, for example.

In addition, compounds of formula I containing an achiral branched group R or R³ may occasionally be of importance, for example, due to a reduction in the tendency towards crystallization. Branched groups of this type generally do not contain more than one chain branch. Preferred achiral branched groups are isopropyl, isobutyl (=methylpropyl), isopentyl (=3-methylbutyl), isopropoxy, 2-methylpropoxy and 3-methylbutoxy.

Another preferred embodiment of the present invention relates to compounds of formula I wherein R, R¹ or R² is denoting P-(Sp-X)ₙ.

R⁰ in formula I is preferably H or CH₃, in particular H.

The polymerisable group P is selected from CH₂=CW¹-COO-, 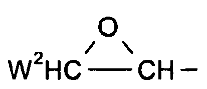 , CH₂=CW²-O, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, OCN- and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

P is particularly preferably an acrylate, methacrylate, vinyl, vinyloxy, epoxy, styrene or propenyl ether group, in particular an acrylate, methacrylate, vinyl or epoxy group.

As spacer group Sp in formula I all groups can be used that are known for this purpose to the skilled in the art. Sp is preferably a straight chain or branched alkylene group having 1 to 20 C atoms, in particular 1 to 12 C atoms, in which, in addition, one or more non-adjacent CH₂ groups may be replaced by -O-, -S-, -NR⁰-, -CO-, -OCO-, -S-CO-, -O-COO-, -CO-S-, -CO-O-, -CH(halogen)-, -CH(CN)-, -CH(OH)-, -(CF₂)ₓ-, -(CD₂)ₓ-, -CH=CH-, -CF=CF-, -CH=CF- or -C≡C-, with x being an integer from 1 to 12, and in which one or more H atoms may be replaced by halogen, CN or OH.

Typical spacer groups are for example -(CH₂)_{y}-, -(CH₂CH₂O)_{z}-CH₂CH₂-, -CH₂CH₂-S-CH₂CH₂- or -CH₂CH₂-NH-CH₂CH₂-, with y being an integer from 2 to 12 and p being an integer from 1 to 3.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyliminoethylene, 1-methylalkylene, ethenylene, propenylene and butenylene for example.

Especially preferred are inventive compounds of formula I wherein Sp is denoting alkylene or alkylene-oxy with 2 to 8 C atoms. Straight-chain groups are especially preferred.

In another preferred embodiment of the invention the compounds of formula I comprise at least one spacer group Sp that is a chiral group of formula V: 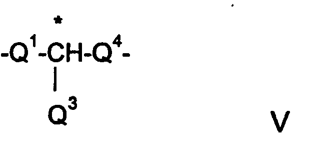 wherein
Q¹ and Q³ have the meanings given in formula IV, and
Q⁴ is an alkylene or alkylene-oxy group with 1 to 10 C atoms or a single bond, being different from Q¹.

In case Q¹ in formula V is an alkylene-oxy group, the O atom is preferably adjacent to the chiral C atom.

Throughout this text, halogen is preferably F or Cl.

The inventive compounds can be synthesized according to or in analogy to methods which are known per se, as described in the literature (for example in the standard works such as Houben-Weyl, Methoden der Organischen Chemie [Methods of Organic Chemistry], Georg-Thieme-Verlag, Stuttgart), to be precise under reaction conditions which are known and suitable for said reactions. Use may also be made here of variants which are known per se, but are not mentioned here. Further methods for preparing the inventive compounds can be taken from the examples.

The inventive compounds can be used in a liquid crystal mixture for liquid crystal displays. Thus, another object of the invention is a liquid crystalline mixture comprising at least one compound of formula I.

Many of the inventive compounds are characterized by a good solubility in liquid crystalline host mixtures, and can be added as dopants to liquid crystalline hosts in high amounts without significantly affecting the phase behaviour and electrooptical properties of the mixture. Undesired spontaneous crystallization at low temperatures is thereby reduced and the operating temperature range of the mixture can be broadened.

A liquid crystalline mixture according to the invention comprises preferably 0.1 to 30 %, in particular 1 to 25 % and very particularly preferably 2 to 15 % by weight of compounds of formula I.

A liquid crystalline mixture according to the invention preferably comprises 1 to 3 chiral compounds of formula I.

In a preferred embodiment of the invention the liquid crystalline mixture is consisting of 2 to 25, preferably 3 to 15 compounds, at least one of which is a compound of formula I. The other compounds are preferably low molecular weight liquid crystalline compounds selected from nematic or nematogenic substances, for example from the known classes of the azoxybenzenes, benzylidene-anilines, biphenyls, terphenyls, phenyl or cyclohexyl benzoates, phenyl or cyclohexyl esters of cyclohehexanecarboxylic acid, phenyl or cyclohexyl esters of cyclohexylbenzoic acid, phenyl or cyclohexyl esters of cyclohexylcyclohexanecarboxylic acid, cyclohexylphenyl esters of benzoic acid, of cyclohexanecarboxylic acid and of cyclohexylcyclohexanecarboxylic acid, phenylcyclohexanes, cyclohexylbiphenyls, phenylcyclohexylcyclohexanes, cyclohexylcyclohexanes, cyclohexylcyclohexenes, cyclohexylcyclohexylcyclohexenes, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexylpyridazines, phenyl- or cyclohexyldioxanes, phenyl- or cyclohexyl-1,3-dithianes, 1,2-diphenyl-ethanes, 1,2-dicyclohexylethanes, 1-phenyl-2-cyclohexylethanes, 1-cyclohexyl-2-(4-phenylcyclohexyl)-ethanes, 1-cyclohexyl-2-biphenyl-ethanes, 1-phenyl2-cyclohexylphenylethanes, optionally halogenated stilbenes, benzyl phenyl ether, tolanes, substituted cinnamic acids and further classes of nematic or nematogenic substances. The 1,4-phenylene groups in these compounds may also be laterally mono- or difluorinated.

The liquid crystalline mixture of this preferred embodiment is based on the achiral compounds of this type.

The most important compounds that are posssible as components of these liquid crystalline mixtures can be characterized by the following formula

R'-L'-G'-E-R"

wherein L' and E, which may be identical or different, are in each case, independently from one another, a bivalent radical from the group formed by -Phe-, -Cyc-, -Phe-Phe-, -Phe-Cyc-, -Cyc-Cyc-, -Pyr-, -Dio-, -B-Phe- and -B-Cyc- and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl abd B is 2-(trans-1,4-cyclohexyl)ethyl, pyrimidine-2,5-diyl, pyridine-2,5-diyl or 1,3-dioxane-2,5-diyl.

G' in these compounds is selected from the following bivalent groups -CH=CH-, -N(O)N-, -CH=CY-, -CH=N(O)-, -C≡C-, -CH₂-CH₂-, -CO-O-, -CH₂-O-, -CO-S-, -CH₂-S-, -CH=N-, -COO-Phe-COO- or a single bond, with Y being halogen, preferably chlorine, or -CN.

R' and R" are, in each case, independently of one another, alkyl, alkenyl, alkoxy, alkenyloxy, alkanoyloxy, alkoxycarbonyl or alkoxycarbonyloxy with 1 to 18, preferably 3 to 12 C atoms, or alternatively one of R' and R" is F, CF₃, OCF₃, Cl, NCS or CN.

In most of these compounds R' and R" are, in each case, independently of each another, alkyl, alkenyl or alkoxy with different chain length, wherein the sum of C atoms in nematic media generally is between 2 and 9, preferably between 2 and 7.

Many of these compounds or mixtures thereof are commercially available. All of these compounds are either known or can be prepared by methods which are known per se, as described in the literature (for example in the standard works such as Houben-Weyl, Methoden der Organischen Chemie [Methods of Organic Chemistry], Georg-Thieme-Verlag, Stuttgart), to be precise under reaction conditions which are known and suitable for said reactions. Use may also be made here of variants which are known per se, but are not mentioned here.

A preferred use of the inventive compounds is the preparation of polymerizable liquid crystalline mixtures, anisotropic polymer gels and anisotropic polymer films, in particular oriented polymer films that exhibit a pattern of different regions with different orientation as describe above.

Patterned polymer films can be used for example as optical elements like colour filters or polarization beam splitters, alignment layers, security markings or anisotropic membranes for the permeation of gases or fluids. Furthermore, the inventive polymers and polymer gels can eb used in optical information storage devices or nonlinear optics.

For the preparation of anisotropic polymer gels or oriented polymer films, the liquid crystalline mixture should comprise at least one polymerizable compound, preferably a polymerizable mesogenic compound.

Thus, another object of the invention are polymerizable liquid crystalline mixtures comprising at least two compounds, at least one of which is a compound of formula I and at least one of which is a polymerizable compound. The polymerizable compound can be said at least one compound of formula I or an additional compound.

Examples of suitable polymerizable mesogenic compounds that can be used as co-components in the polymerizable mixture are disclosed for example in WO 93/22397; EP 0,261,712; DE 195,04,224; WO 95/22586 and WO 97/00600. The compounds disclosed in these documents, however, are to be regarded merely as examples that shall not limit the scope of this invention. Preferably the polymerizable mixture comprises at least one polymerizable mesogenic compound having one polymerizable functional group and at least one polymerizable mesogenic compound having two or more polymerizable functional groups.

Examples of especially useful monoreactive chiral and achiral polymerizable mesogenic compounds are shown in the following list of compounds, which should, however, be taken only as illustrative and is in no way intended to restrict, but instead to explain the present invention: 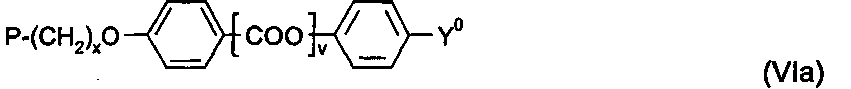 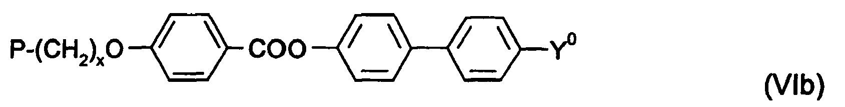 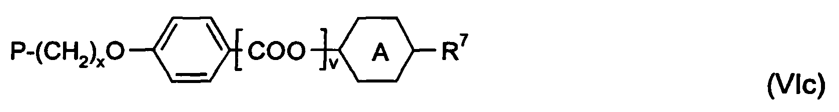 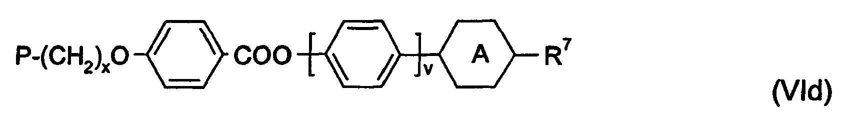 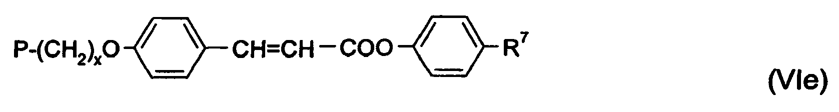 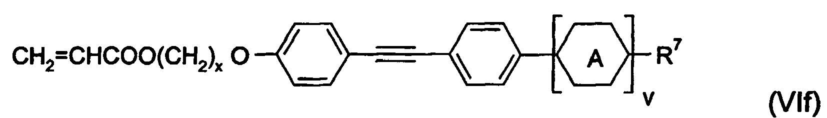 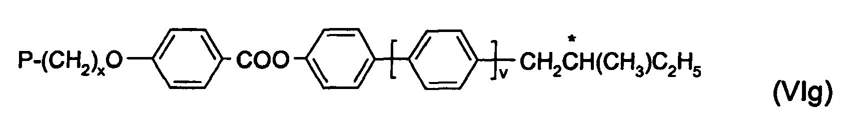 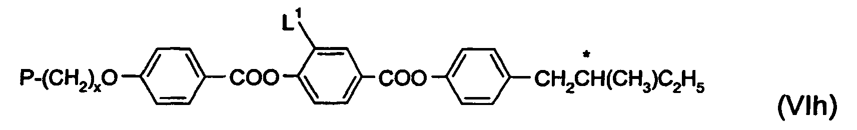 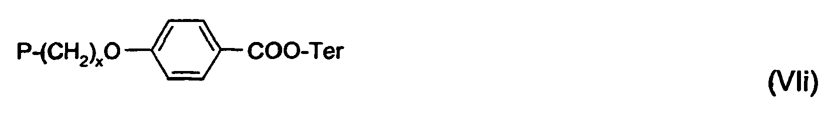 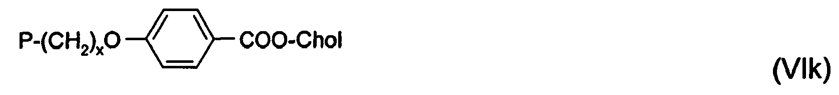 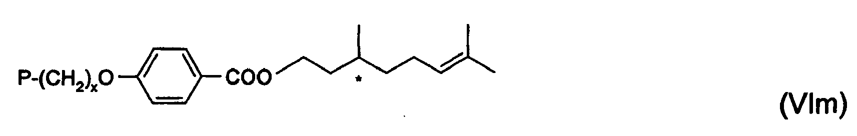 wherein, P has one of the meanings given above, x is an integer from 1 to 12 , A is 1,4-phenylene or 1,4-cyclohexylene, v is 0 or 1, Y⁰ is a polar group, R⁷ is an unpolar alkyl or alkoxy group, Ter is a terpenoid radical like e.g. menthyl, Chol is a cholesteryl group, and L¹ and L² are each independently H, F, Cl, CN, OH, NO₂ or an optionally halogenated alkyl, alkoxy or carbonyl group with 1 to 7 C atoms.

The polar group Y⁰ is preferably CN, NO₂, halogen, OCH₃, OCN, SCN, COR⁸, COOR⁸ or a mono- oligo- or polyfluorinated alkyl or alkoxy group with 1 to 4 C atoms. R⁸ is optionally fluorinated alkyl with 1 to 4, preferably 1 to 3 C atoms. Especially preferably the polar group Y⁰ is selected of F, Cl, CN, NO₂, OCH₃, COCH₃, COC₂H₅, COOCH₃, COOC₂H₅, CF₃, C₂F₅, OCF₃, OCHF₂, and OC₂F₅, in particular F, Cl, CN, OCH₃ and OCF₃.

The unpolar group R⁷ is preferably an alkyl group with 1 or more, preferably 1 to 15 C atoms or an alkoxy group with 2 or more, preferably 2 to 15 C atoms.

Examples of useful direactive chiral and achiral polymerizable mesogenic compounds are shown in the following list of compounds, which should, however, be taken only as illustrative and is in no way intended to restrict, but instead to explain the present invention 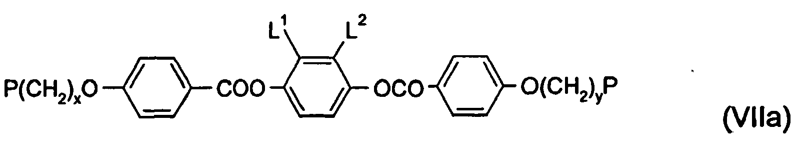 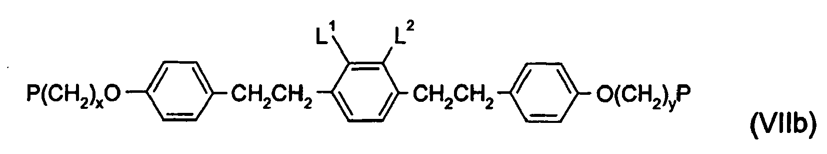 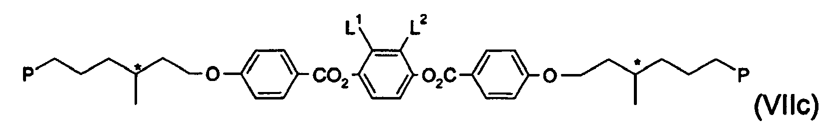 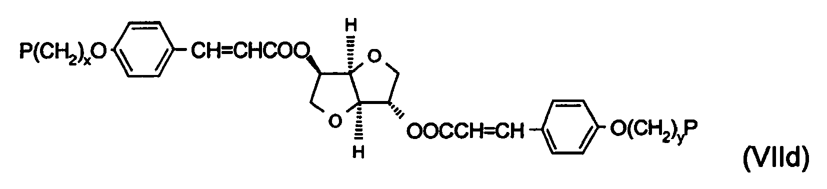 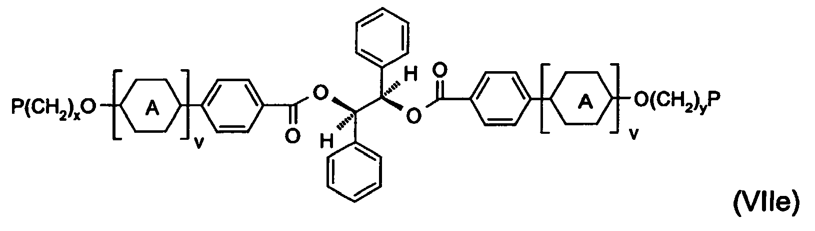 wherein P, x, A, L¹ and L² have one of the meanings given above and y is an integer from 1 to 12 the same as or different from x.

The mono- and difunctional polymerizable mesogenic compounds of above formulae VI and VII can be prepared by methods which are known per se and which are described in the documents cited above and, for example, in standard works of organic chemistry such as, for example, Houben-Weyl, Methoden der organischen Chemie, Thieme-Verlag, Stuttgart.

In a preferred embodiment of the invention the polymerizable liquid crystalline mixtures comprise at least one inventive compound of formula I, at least one monofunctional compound of formulae Vla-VIm and at least one bifunctional polymerizable compound of formulae VIIa-VIIe.

In another preferred embodiment the polymerizable liquid crystalline mixtures comprise at least one inventive compound of formula I and at least two monofunctional compounds of formulae Vla-Vlm.

Another object of the invention is an anisotropic polymer film with an oriented chiral liquid crystalline phase obtainable by (co)polymerizing a liquid crystalline mixture comprising at least one compound of formula I and at least one polymerizable mesogenic compound preferably selected of formula Vla-Vlm and VIIa-VIIe and/or at least one polymerizable compound of formula I.

The preparation of an anisotropic polymer film from a polymerizable mixture is generally described for example in D.J. Broer, et al., Angew. Makromol. Chem. 183, (1990), 45-66. The cholesteric polymerizable mixture is coated onto a substrate, aligned into uniform planar orientation, and polymerized in situ by exposure to heat or actinic radiation, thereby fixing the uniform alignment. Alignment and curing are carried out in the liquid crystal phase of the polymerizable mixture.

Actinic radiation means irradiation with light, like UV light, IR light or visible light, irradiation with X-rays or gamma rays or irradiation with high energy particles, such as ions or electrons. As a source for actinic radiation for example a single UV lamp or a set of UV lamps can be used. Another possible source for actinic radiation is a laser, like e.g. a UV laser, an IR laser or a visible laser.

For example, when photopolymerizing by means of UV light, a photoinitiator can be used that decomposes under UV irradiation to produce free radicals or ions that start the polymerization reaction. It is also possible to use a cationic photoinitiator, when curing polymerizable mesogens with for example vinyl and epoxide reactive groups, that photocures with cations instead of free radicals. As a photoinitiator for radical polymerization for example the commercially available Irgacure 651, Irgacure 184, Darocure 1173 or Darocure 4205 (all from Ciba Geigy AG) can be used, whereas in case of cationic photopolymerization the commercially available UVI 6974 (Union Carbide) can be used. Preferably the polymerizable liquid crystalline mixtures comprising polymerizable compounds of formula I and/or polymerizable mesogenic compounds of formulae VI and VII additionally comprise 0.01 to 10 %, in particular 0.05 to 8 %, very preferably 0.1 to 5 % by weight of a photoinitiator, especially preferably a UV-photoinitiator.

Preferably polymerization is carried out under an atmosphere of inert gas, preferably under a nitrogen atmosphere.

As a substrate for example a glass or quarz sheet as well as a plastic film or sheet can be used. It is also possible to put a second substrate on top of the coated mixture prior to, during and/or after polymerization. The substrates can be removed after polymerization or not. When using two substrates in case of curing by actinic radiation, at least one substrate has to be transmissive for the actinic radiation used for the polymerization. Isotropic or birefringent substrates can be used. In case the substrate is not removed from the polymerized film after polymerization, preferably isotropic substrates are used.Preferably at least one substrate is a plastic substrate such as for example a film of polyester such as polyethyleneterephthalate (PET), of polyvinylalcohol (PVA), polycarbonate (PC) or triacetylcellulose (TAC), especially preferably a PET film or a TAC film. As a birefringent substrate for example an uniaxially stretched plastic film can be used. For example PET films are commercially available from ICI Corp. under the trade name Melinex.

The polymerizable mixture is preferably coated as a thin layer on a substrate or between substrate, and aligned in its liquid crystal phase, e.g. the nematic or smectic phase, to give a planar orientation, i.e. wherein the optical axis of the liquid crystal material is parallel to the plane of the layer. Planar orientation can be achieved for example by shearing the mixture, e.g. by means of a doctor blade. It is also possible to apply an alignment layer, for example a layer of rubbed polyimide or sputtered SiOₓ, on top of at least one of the substrates. Alternatively, a second substrate is put on top of the coated material. In this case, the shearing caused by putting together the two substrates is sufficient to give good alignment. It is also possible to apply an electric or magnetic field to the coated mixture.

In some cases it is of advantage to apply a second substrate not only to aid alignment of the polymerizable mixture but also to exclude oxygen that may inhibit the polymerization. Alternatively curing can be carried out under an atmosphere of inert gas. However, curing in air is also possible using suitable photoinitiators and high lamp power. When using a cationic photoinitiator oxygen exclusion most often is not needed, but water should be excluded.

An inventive polymerizable liquid crystalline mixture for the preparation of anisotropic polymer films comprises preferably 0.1 to 35 %, in particular 0.5 to 15 % and very particularly preferably 0.5 to 5 % by weight of one or more polymerizable chiral compounds of formula I. Polymerizable liquid crystalline mixtures are preferred that comprise 1 to 3 chiral compounds of formula I.

The inventive polymerizable liquid crystalline mixtures can additionally comprise one or more other suitable components such as, for example, catalysts, sensitizers, stabilizers, inhibitors, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Preferably the inventive polymerizable mixture comprises a stabilizer that is used to prevent undesired spontaneous polymerization for example during storage of the composition. As stabilizers in principal all compounds can be used that are known to the skilled in the art for this purpose. These compounds are commercially available in a broad variety. Typical examples for stabilizers are 4-ethoxyphenol or butylated hydroxytoluene (BHT).

It is also possible, in order to increase crosslinking of the polymers, to add a non mesogenic compound with two or more polymerizable functional groups, preferably in an amount of up to 20% by weight, to the polymerizable mixture alternatively or additionally to multifunctional mesogenic polymerizable compounds. Typical examples for difunctional non mesogenic monomers are alkyldiacrylates or alkyldimethacrylates with alkyl groups of 1 to 20 C atoms. Typical examples for non mesogenic monomers with more than two polymerizable groups are trimethylpropanetrimethacrylate or pentaerythritoltetraacrylate.

Polymerization of inventive compositions comprising compounds with only one polymerizable functional group leads to linear polymers, whereas in the presence of compounds with more than one polymerizable functional group crosslinked polymers are obtained.

For the preparation of anisotropic polymer gels, the liquid crystalline mixtures can be polymerized in situ as described above, however, in this case alignment of the polymerizable mixture is not always necessary.

Due to the presence of a photoisomerizable group in the compounds of formula I, the orientation of the inventive compounds and liquid crystalline mixtures can be changed by photoirradiation. Photoirradiation can be achieved for example with irradiation by UV light or other high energy sources such as lasers.

The compounds of formula I and the liquid crystalline mixtures and polymer s comprising them are useful in optical and electrooptical devices like liquid crystal displays or projection systems, patterned films and optical elements like polarizers, retardation films, compensators, colour filters, holographic elements or polarization beam splitters, as sensitizers, for photoswitching, in anisotropic membranes for the permeation of gases or fluids, adhesives, synthetic resins with anisotropic mechanical properties, cosmetic or pharmaceutical compositions, UV absorbers and sunscreens, diagnostics or liquid crystal pigments, for decorative and security applications, in nonlinear optics, optical information storage or as dopants.

The compounds of formula I and liquid crystalline mixtures, liquid crystal polymers or liquid crystal pigments comprising them are also suitable for use in cosmetic and pharmaceutical compositions, for example as UV absorbers or sunscreens for the protection of human skin or hair, in particular protection against UV-A and UV-B-radiation, as described for example in JP 04-134043.

A liquid crystalline mixture, liquid crystal polymer or liquid crystal pigment comprising a compound of formula I and reflecting UV light, in particular of a wavelength of 200 to 400 nm, is another object of the invention. Another object is a cosmetic composition, in particular a cosmetic or pharmaceutical composition for protection of human skin or hair, comprising as UV-filter a compound of formula I or a liquid crystalline mixture, liquid crystal polymer or liquid crystal pigment comprising a compound of formula I and reflecting UV light, in particular in a wavelength range of 200-440 nm, especially 280-400 nm, 200-230 nm (UV-C) and 280-330 nm (UV-B).

Furthermore, the compounds of formula I are particularly suitable for the preparation of patterned films, for example by the following method:

A thin layer of a polymerizable nematic mixture comprising an inventive photoisomerizable compound of formula I is coated as a thin film onto a substrate and aligned into planar orientation as described above. If the coated film is exposed to photoradiation of a suitable wavelength, the compound of formula I shows *EZ* isomerization if the wavelength of radiation is selected accordingly, e.g. between 300 and 400 nm. This causes a disruption of the planar nematic orientation in the exposed parts of the film, and can even lead to a radiation-induced nematic-isotropic phase transition in the exposed region. Thereby the birefringence is changing in the exposed parts. The partially or totally unoriented state can be fixed by subsequent in-situ polymerization, e.g. by thermal or photocuring.

If only a part of the film is exposed to photoradiation, e.g. by irradiation through a photomask that is applied on top of the coated film, the nematic orientation can be destroyed and the unoriented state subsequently be fixed by thermal or photocuring of the exposed parts. Afterwards the highly oriented state in the previously unexposed parts of the film is also fixed by thermal or photocuring. The sequence of these steps may also be changed, e.g. first fixing of the oriented parts by photocuring through a photomask, and then photoisomerization and fixing of the remaining parts.

The above described methods can also be used to prepare patterned films with chiral liquid crystalline structure, such as e.g. cholesteric or chiral smectic C liquid crystal films.

For example cholesteric liquid crystal films with planar alignment show selective reflection of visible light that is circularly polarized, caused by interaction of incident light with the helically twisted structure of the cholesteric material. If one or more chiral compounds of formula I are added e.g. to nematic or cholesteric polymerizable liquid crystal mixture, photoisomerization of the chiral compounds of formula I leads to a change of their chirality and thus to a change of the helical pitch of the mixture, which is fixed by polymerization. As the pitch is directly proportional to the reflection wavelength of the cholesteric material, patterned cholesteric films having regions of different reflection wavelength can be obtained by using photomask technique as described above.

Such films are suitable for example as colour filters in optical or electrooptical devices like liquid crystal displays or projectors. They can also be used for security markings, e.g. to identify or prevent falsification of credit cards, passports, bank notes or other documents of value, for decorative coatings or the preparation of liquid crystal pigments.

Furthermore, cholesteric reflective films exhibiting a change of the pitch in vertical direction, i.e. perpendicular to the film plane, can be prepared. They can be used as broadband reflective polarizers having a broad bandwidth of the reflected wavelength band.

The preparation of patterned cholesteric films is described for example in WO 00/34808 and in P. van de Witte et al., J. Mater. Chem. 9 (1999), 2087-2094, the entire disclosure of which is incorporated into this application by way of reference.

A broadband reflective cholesteric film can e.g. be prepared as follows: A layer of a cholesteric or nematic mixture with planar orientation comprising a chiral photoisomerizable compound of formula I additionally comprises a dye having an absorption maximum at the wavelength where the isomerizable compound shows photoisomerization. For example, the mixture may comprise an isomerizable compound showing isomerization at a wavelength in the UV range together with a UV dye. If the mixture is exposed to UV radiation as described above, the dye will create a gradient in UV light intensity throughout the thickness of the layer. As a consequence, the isomerization is faster at the top of the layer than at the bottom and a pitch gradient is created, leading to a broadening of the reflected wavelength band. The pitch gradient and reflection bandwidth can be controlled for example by varying the film thickness, irradiation time, radiation dose and/or the concentration of the UV dye and the photoisomerizable compound. If the cholesteric mixture comprises one or more polymerizable components, the structure of the film can be fixed by in-situ polymerization.

It is also possible to prepare cholesteric liquid crystal films wherein the pitch varies in horizontal or vertical direction by using an achiral isomerizable compound of formula I together with a cholesteric liquid crystal mixture. As described above, the achiral isomerizable compound changes its shape e.g. due to *EZ* isomerization under photoirradiation, causing a change of the order parameter and thus of the pitch in the cholesteric mixture in the irradiated regions. If the mixture contains a polymerizable compound, the region with different pitch can be fixed by subsequent in-situ polymerization. Thereby, patterned cholesteric films having regions of different reflection wavelength can be obtained by using photomask technique as described above. Alternatively, a broadband cholesteric film with the pitch varying in vertical direction can be obtained by using e.g. a UV dye that creates an intensity gradient of the photoradiation in vertical direction as described above.

From the foregoing description, one skilled in the art can easily ascertain the essential characteristics of this invention, and without departing from the spirit and scope thereof, can make various changes and modifications of the invention to adapt it to various usages and conditions.

Without further elaboration, it is believed that one skilled in the art can, using the preceding description, utilize the present invention to its fullest extent. The following examples are, therefore, to be construed as merely illustrative and not limitative of the remainder of the disclosure in any way whatsoever.

In the foregoing and in the following examples, unless otherwise indicated, all temperatures are set forth uncorrected in degrees Celsius and all parts and percentages are by weight.

The following abbreviations are used to illustrate the liquid crystalline phase behaviour of the compounds: K = crystalline; N = nematic; S = smectic; N*, Ch = chiral nematic or cholesteric; I = isotropic. The numbers between these symbols indicate the phase transition temperatures in degree Celsius. Furthermore, mp. is the melting point, Δn is the birefringence at 589 nm and 20 °C and Δε is the dielectric anisotropy at 20 °C. C* in a chemical formula denotes a chiral C atom. DCM is dichloromethane.

"Conventional workup" means: water is added if necessary, the mixure is extracted with methylene chloride, diethyl ether or toluene, the phases are separated, the organic phase is dried and concentrated by evaporation, and the product is purified by crystallization and/or chromatography.

### Examples

### Example 1

Compound (1) was prepared according to the following reaction scheme

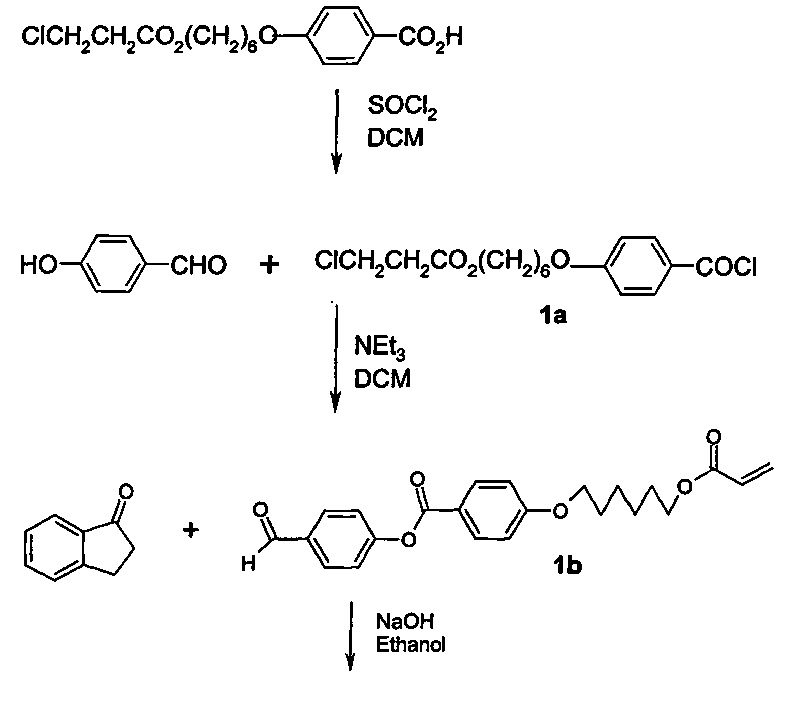

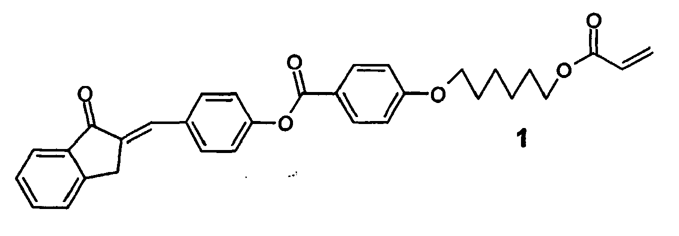

### 3-Chloro-propionic acid 6-(4-chlorocarbonvl-phenoxv)-hexyl ester (1a)

HHBA-3-chloropropionate (15.0 g, 45.6 mmol) was stirred with thionyl chloride (4.4 ml, 60.3 mmol, 1.3 equiv.) and a catalytic amount of N-methyl pyrrolidone under reflux in DCM for 16 hours. The mixture was cooled and evaporated to dryness to leave an oily residue. Yield = 16.2 g, 100%. The product was used without further purification in the next step.

### 4-(6-Acryloyloxy-hexyloxy)-benzoic acid 4-formyl-phenyl ester (1b)

Acid chloride (15.5 g, 45.6 mmol), 4-hydroxybenzaldehyde (5.6 g, 45.6 mmol) and triethylamine (25.4 ml, 183 mmol) were stirred in DCM at 35°C overnight. The mixture was cooled to room temperature, the DCM layer was washed with water, then dilute hydrochloric acid then dried (Na₂SO₄). Evaporation of the solvent left a pale pink solid residue. This was used without further purification.

### Compound (1)

1-Indanone (2.31 g, 0.017 mol) was dissolved in ethanol (12 ml), and was added to sodium hydroxide (0.23 g, 0.005 mol) dissolved in water (4 ml) and ethanol (1.5 ml) and was left to stir for 20 min. 2 (6.76 g, 0.017 mol) in ethanol (20 ml) was added to the mixture and it was left to stir. After 16 h, a solid precipitate was removed by vacuum filtration and was washed with cold ethanol (2 x 20 ml).
Yield 0.72 g, 8% ¹H NMR and ¹³C NMR gave expected signals.
Mass spec. ES+, 533.1 (M + Na⁺), 1732.15 (2M + Na⁺).

Transition Temperatures:
K-124-131-I (first heat)
I-130-N-109-K (first cool)
K-131-I (second heat)
I-123-N-110-K (second cool)

### Example 2-Comparative Example

Compound (2) was prepared according to the following reaction scheme

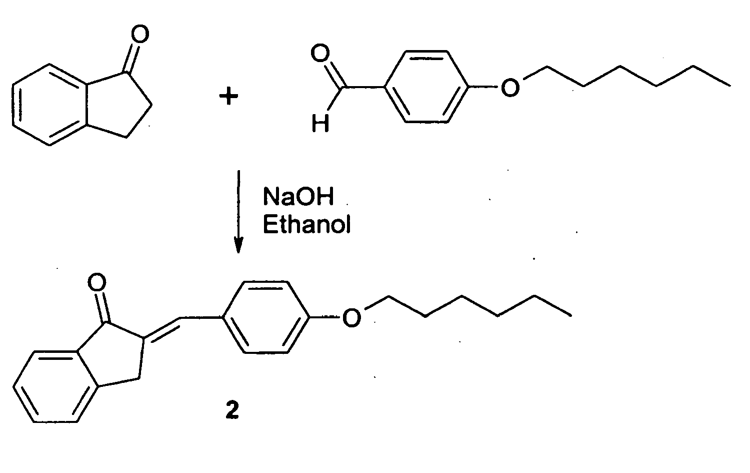

### 2-(4-Hexyloxy-benzylidene)-indan-1-one (2)

1-Indanone (4.17 g, 0.032 mol) was dissolved in ethanol (20 ml), and was added to sodium hydroxide (0.44 g, 0.011 mol) dissolved in water (8 ml) and ethanol (3 ml) and was left to stir for 20 min. 4-(Hexyloxy)benzaldehyde (6.4 ml, 0.031 mol), dissolved in ethanol (35 ml), was added to the mixture and was stirred for 4 hours. The product was isolated by vacuum filtration and was washed with cold ethanol (2 x 20 ml) and was dried in a vacuum oven. Yield = 8.47 g, 85%. ¹H NMR and ¹³C NMR gave expected signals.
Melting point 101°C, (K-I, ΔH= 8399 cal mol⁻¹).

### Example 3

### Photoisomerisation of (1) in a polymerizable liquid crystal mixture

The following polymerizable mixture was prepared:

| | |
|---|---|
| Compound (1) | 10% |
| Compound (A) | 20 % |
| Compound (B) | 70 % |

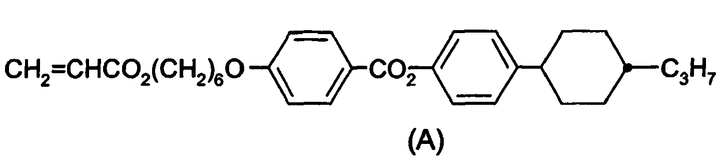

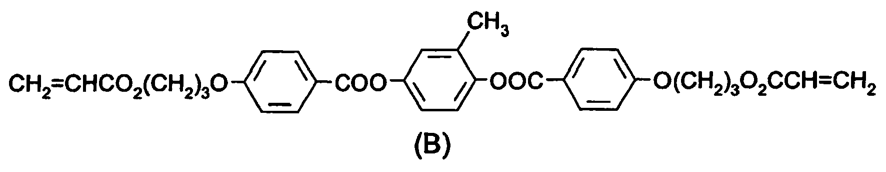

Compound (A) is described in WO 98/00428. Compound (B) can be prepared as described in D.J.Broer et al., Makromol. Chem. 190, 3201-3215 (1989).

The above mixture was dissolved in THF (20%w/w of mixture in THF). A film of the above mixture was made by spin coating the solution onto rubbed PI coated glass at 3000rpm. The film was slightly opaque when viewed in transmission. The film was then annealed at 100°C for 30 seconds, whereupon the alignment of the film improved, and the film became transparent. When viewed between crossed polariser, the film had maximum transmission when the rubbing direction was at 45° to the stretch direction of the polariser, and minimum transmission when the rubbing direction was parallel or perpendicular to the polariser stretch direction. The film was then exposed to 360nm UV light through a plastic mask (black ink on a PET substrate) from a high-pressure mercury lamp at 18mW/cm² for 10 minutes. When the exposed film was viewed between crossed polarisers, with the rubbing direction at 45° to the stretch direction of the polarisers, then the areas which had experience a higher exposure to the UV light showed lower transmission compared to the unexposed areas.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and can make various changes and modifications of the invention to adapt it to various conditions and usages.

## Claims

1. Polymerizable liquid crystalline mixture comprising at least one compound of formula I, and comprising at least one polymerizable compound, which can be a compound of formula I or an additional polymerizable compound 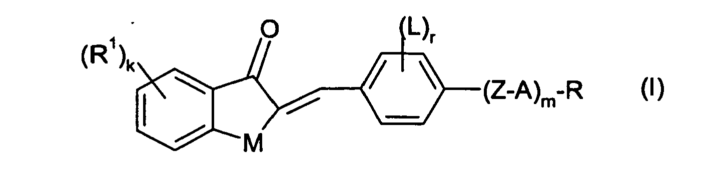 wherein
M is (CH₂)ᵢ, wherein one CH₂ group may also be replaced by NR⁰, O or S,
i is 1, 2, 3 or 4,
R⁰ is H or alkyl with 1 to 4 C atoms,
L is halogen, CN, SCN, NO₂, SF₅ or an alkyl, alkoxy, alkylcarbonyl or alkoxycarbonyl group with 1 to 4 C atoms, wherein one or more H atoms may be substituted with F or Cl.
r is 0, 1, 2, 3 or 4,
R¹ is in each case independently OH, O-B, OCH₂B or has one of the meanings of R given below,
k is 0, 1, 2, 3 or 4,
A and B are independently of each other an aromatic or alicyclic group with 4 to 12 C-atoms,
Z is in each case independently -O-, -S-, -CO-, -COO-,OCO-, -CO-NR⁰-, -NR⁰-CO-, -CH₂CH₂-, -CF₂CF₂-,CH₂CF₂-, -OCH₂-, -CH₂O-, -SCH₂-, -CH₂S-, -CF₂O-,OCF₂-, -CH=CH-, -CF=CF-, -CH=CF-, -(CH₂)₄-, -CH=CH-COO-, -OCO-CH=CH-, -OOC-CHR⁰⁰-OOC-, -COO-CHR⁰⁰-COO-, -C≡C- or a single bond,
R⁰⁰ is straight chain or branched alkyl or alkoxy with 1 to 8 C atoms or phenyl that is optionally mono- or polysubstituted by L,
m is 0, 1 or 2,
R is halogen, NO₂, CN, SCN, SF₅, straight chain, branched or cyclic alkyl with 1 to 25 C atoms wherein one or more CH₂ groups can also be replaced by -O-, -S-, -CO-, -NR⁰-, -CH=CH-, -C≡C- in such a manner that O and/or S atoms are not linked directly to one another, and wherein one or more H-atoms can also be replaced by F or Cl or phenyl that is optionally mono- or polysubstituted by L, or denotes P-(Sp-X)ₙ-,
P is a polymerizable group selected from CH₂=CW¹-COO-, 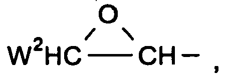 CH₂=CW²-O, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, OCN- and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1,
Sp is a spacer group with 1 to 20 C atoms,
X has one of the meanings of Z, and
n is 0 or 1,
with the proviso that m is 1 or 2 and/or at least one of R and R¹ denotes P-(Sp-X)ₙ-.

2. Mixture according to claim 1, wherein M is NR⁰, O, S, CH₂, (CH₂)₂, NR⁰-CH₂, O-CH₂ or S-CH₂.

3. Mixture according to claim 1 or 2, wherein m is 1 or 2.

4. Mixture according to any of claims 1 to 3, wherein at least one of R and R¹ denotes P-(Sp-X)ₙ-.

5. Mixture according to any of claims 1 to 4, wherein
A is in each case independently 1,4-phenylene in which, in addition, one or more CH groups may be replaced by N, 1,4-cyclohexylene in which, in addition, one or two non-adjacent CH₂ groups may be replaced by O and/or S, 1,3-dioxolane-4,5-diyl, 1,4-cyclohexenylene, piperidine-1,4-diyl, pyrrolidine-2,4-diyl, 1,4-bicyclo-(2,2,2)-octylene, naphthalene-2,6-diyl, decahydro-naphthalene-2,6-diyl, 1,2,3,4-tetrahydronaphthalene-2,6-diyl or indane-2,5-diyl, it being possible for all these groups to be unsubstituted, mono- or polysubstituted with L as defined in formula I.

6. Mixture according to any of claims 1 to 5, wherein the group 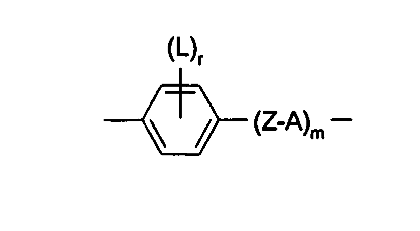 in formula I is selected from the following formulae 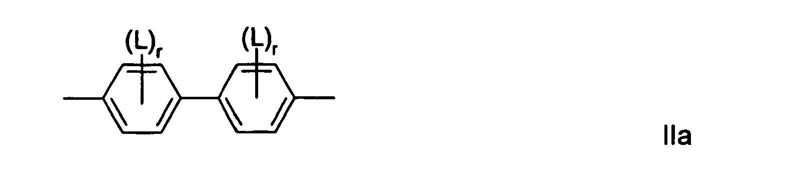 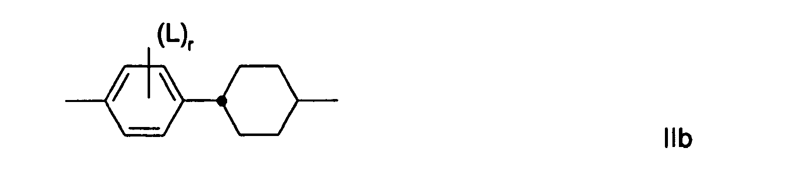 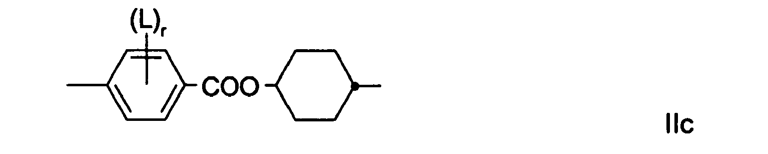 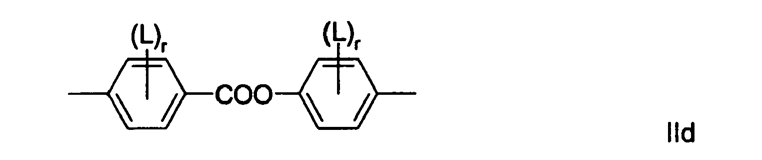 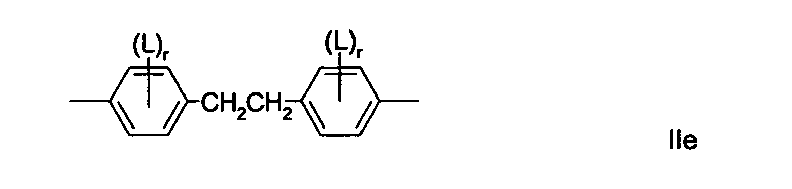 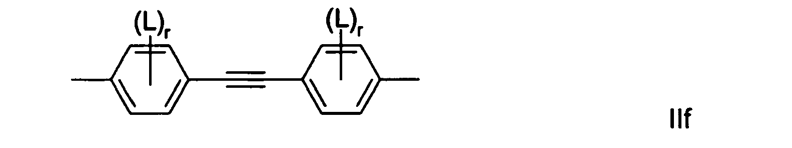 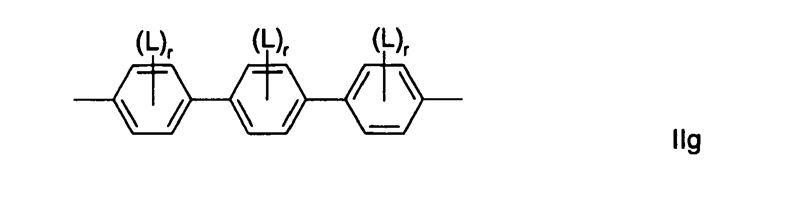 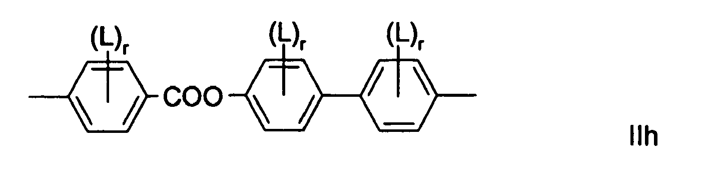 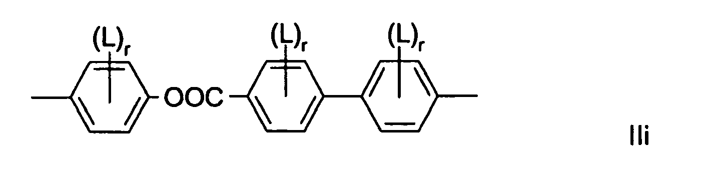 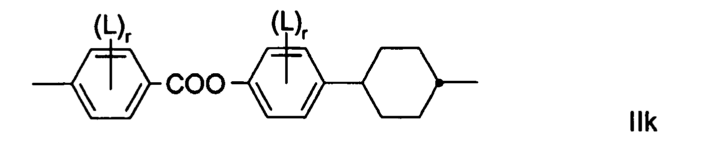 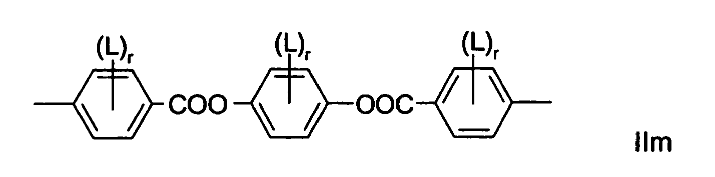 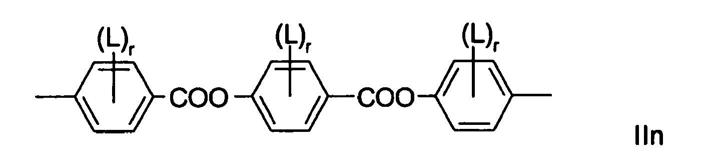 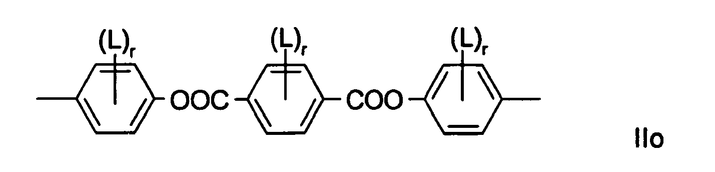 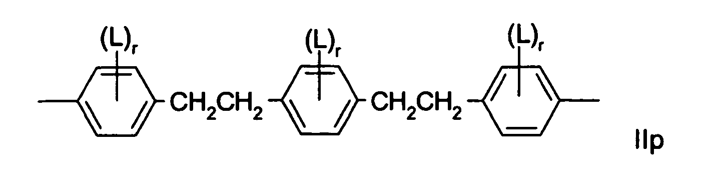 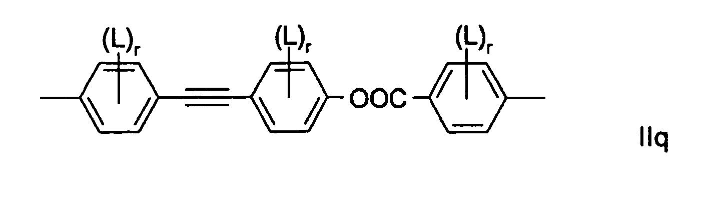 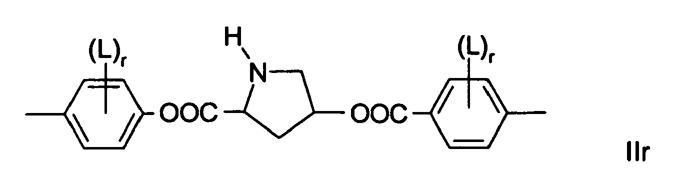 wherein L has the meaning of formula I and r is 0, 1 or 2.

7. Mixture according to any of claims 1 to 6, wherein the compound of formula I is selected of the following subformulae 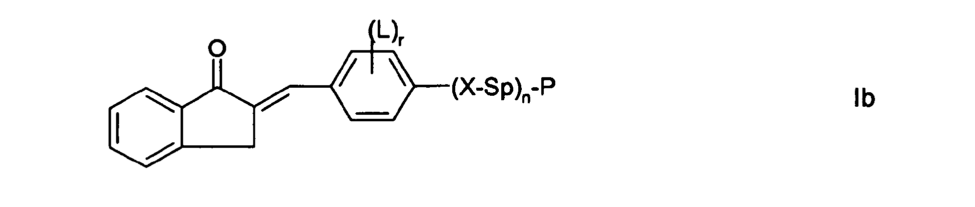 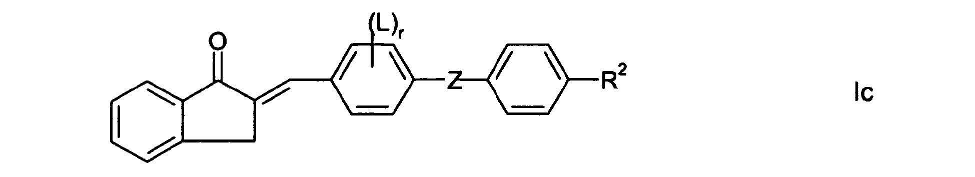 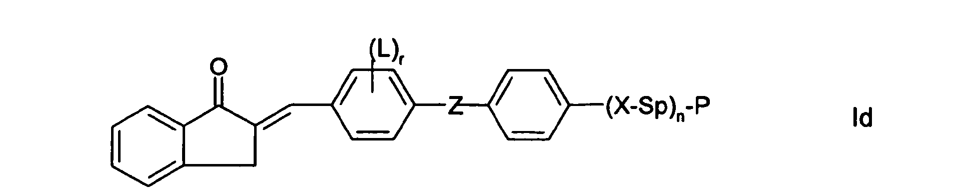 wherein Z, P, Sp, X, L, n and r are as defined in formula I and
R² is H, halogen, NO₂, CN, SCN, SF₅, straight chain, branched or cyclic alkyl with 1 to 25 C atoms wherein one or more CH₂ groups can also be replaced by -0-,S-, -CO-, -NR⁰-, -CH=CH-, -C≡C- in such a manner that O and/or S atoms are not linked directly to one another, and wherein one or more H-atoms can also be replaced by F or Cl.

8. Mixture according to any of claims 1 to 7, wherein P is an acrylate, methacrylate, vinyloxy, epoxy, styrene or propenyl ether group.

9. Mixture according to any of claims 1 to 8, wherein R and/or R¹ is an alkyl or alkoxy group with 1 to 12 C atoms.

10. Mixture according to any of claims 1 to 9, comprising at least one polymerizable compound selected from the following formulae 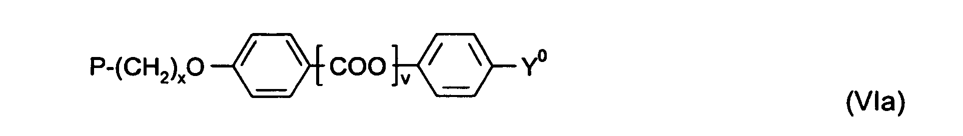 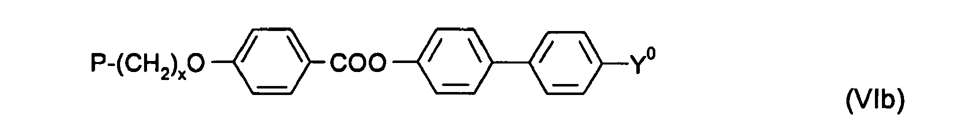 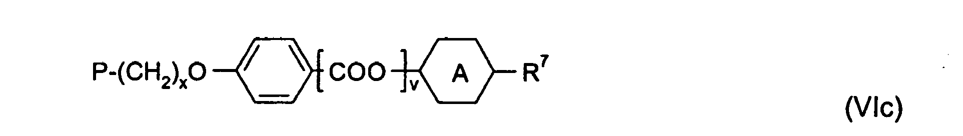 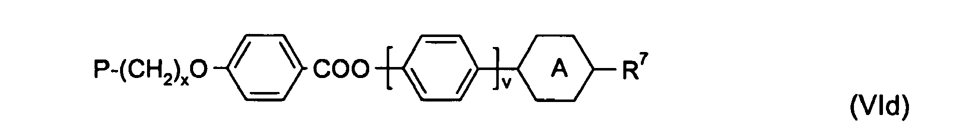 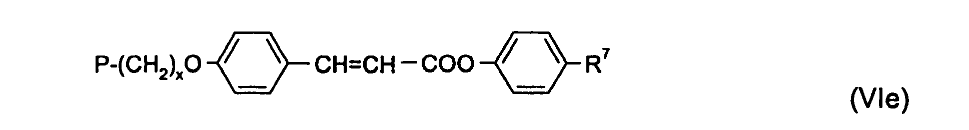 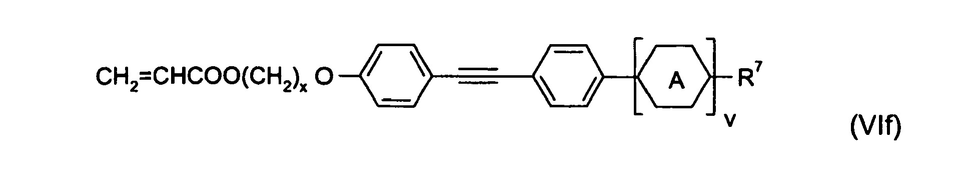 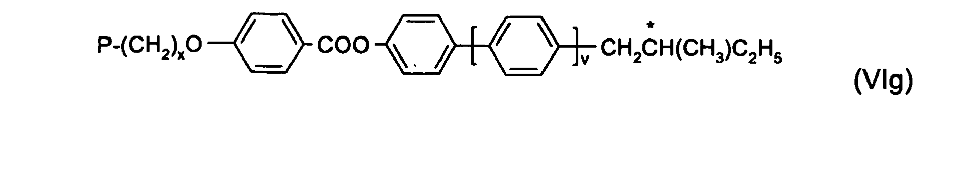 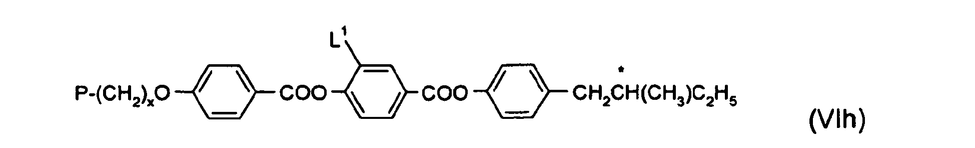 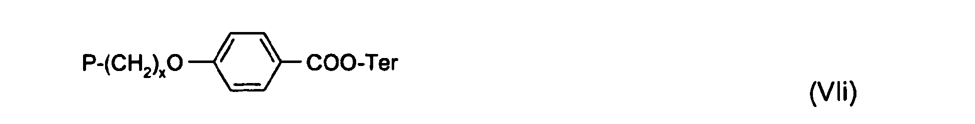 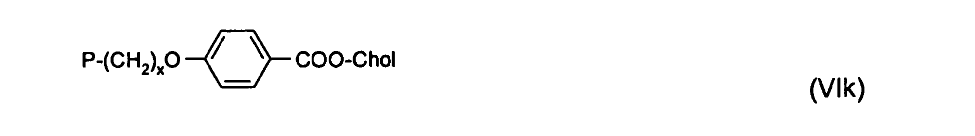 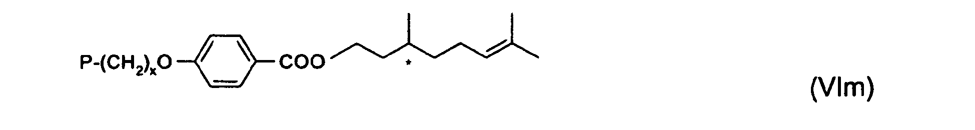 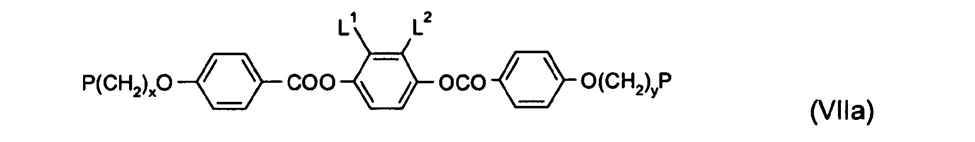 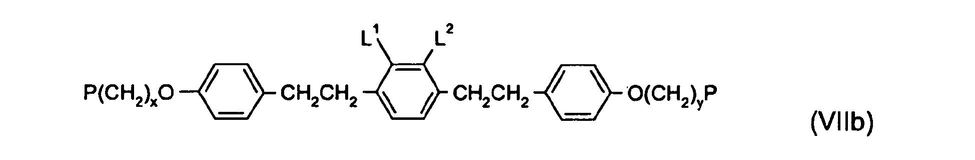 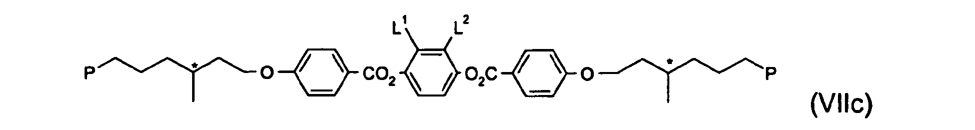 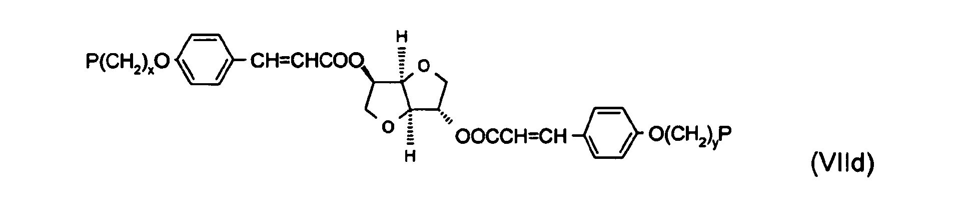 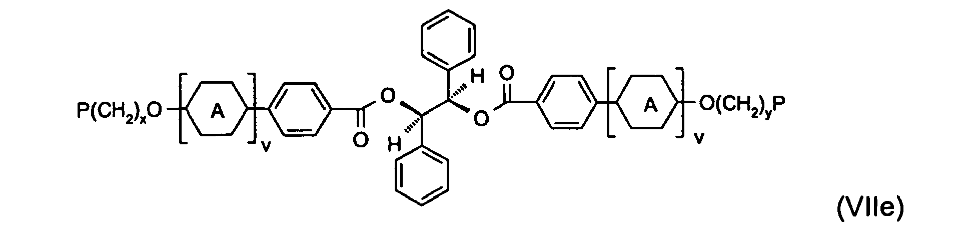 wherein
P has one of the meanings of claim 1,
x is an integer from 1 to 12,
y is an integer from 1 to 12 the same as or different from x,
A is 1,4-phenylene or 1,4-cyclohexylene,
v is 0 or 1,
Y⁰ is CN, NO₂, halogen, OCH₃, OCN, SCN, COR⁸, COOR⁸ or a mono- oligo- or polyfluorinated alkyl or alkoxy group with 1 to 4 C atoms,
R⁸ is optionally fluorinated alkyl with 1 to 4 C atoms,
R⁷ is an alkyl group with 1 to 15 C atoms or an alkoxy group with 2 to 15 C atoms,
Ter is a terpenoid radical,
Chol is a cholesteryl group, and
L¹ and L² are each independently H, F, Cl, CN, OH, NO₂ or an optionally halogenated alkyl, alkoxy or carbonyl group with 1 to 7 C atoms.

11. Linear or crosslinked anisotropic polymer obtainable by polymerizing a mixture according to any of claims 1 to 10.

12. Compound of formula I 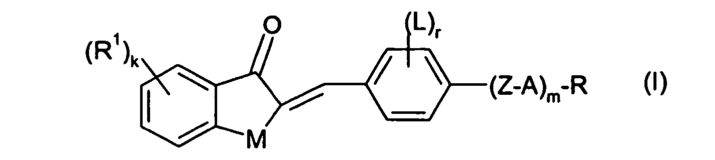 wherein M, R, R¹, A, Z, L, k, m and r are as defined in any of claims 1 to 9, and wherein at least one of R and R¹ denotes P-(Sp-X)ₙ-.

13. Use of photoisomerizable compounds, mixtures and polymers according to claims 1 to 12 in optical and electrooptical devices like liquid crystal displays or projection systems, patterned films and optical elements like polarizers, retardation films, compensators, colour filters, holographic elements or polarization beam splitters, as sensitizers, for photoswitching, in anisotropic membranes for the permeation of gases or fluids, adhesives, synthetic resins with anisotropic mechanical properties, cosmetic or pharmaceutical compositions, UV absorbers and sunscreens, diagnostics or liquid crystal pigments, for decorative and security applications, in nonlinear optics, optical information storage or as dopants.

14. Liquid crystal display comprising a compound, mixture or polymer according to any of claims 1 to 12.

15. Polymer film obtainable by coating a polymerizable mixture according to any of claims 1 to 10 onto a substrate, aligning the mixture into uniform orientation, and polymerizing the mixture, wherein at least a part of the mixture before or during polymerization is exposed to actinic radiation of a wavelength where the compound of formula I shows photoisomerization.

16. Polymer film according to claim 15 showing a pattern of at least two regions having different orientation.

17. Polymer film according to claim 15 showing a pattern of at least two regions having different birefringence.

18. Polymer film according to claim 15 showing a pattern of at least one region with an oriented state and at least one region with a partially or totally unoriented state.

19. Polymer film according to any of claims 15 to 18, **characterized in that** it is a nematic film.

20. Polymer film according to claim 15, **characterized in that** it is a patterned cholesteric film having planar alignment and regions of different reflection wavelength.

21. Polymer film according to claim 15, **characterized in that** it is a broadband reflective cholesteric film.

22. Polymer film according to claim 15, **characterized in that** it is a cholesteric film wherein the pitch varies in horizontal or vertical direction, prepared from an achiral isomerizable compound of formula I together with a cholesteric liquid crystal mixture.

23. Colour filter, security marking, decorative coating or broadband reflective polarizer comprising a polymer film according to any of claims 15 to 22.

## Patentansprüche

1. Polymerisierbare flüssigkristalline Mischung enthaltend mindestens eine Verbindung der Formel I und enthaltend mindestens eine polymerisierbare Verbindung, bei der es sich um eine Verbindung der Formel I oder eine zusätzliche polymerisierbare Verbindung 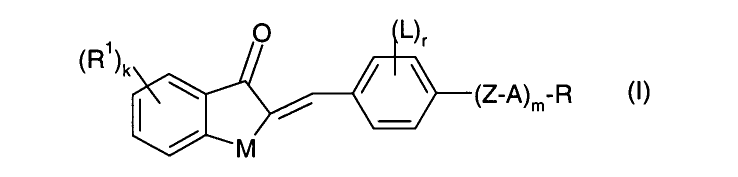 handeln kann, worin
M (CH₂)ᵢ bedeutet, worin eine CH₂-Gruppe auch durch NR⁰, O oder S ersetzt sein kann,
i 1, 2, 3 oder 4 bedeutet,
R⁰ H oder Alkyl mit 1 bis 4 C-Atomen bedeutet,
L Halogen, CN, SCN, NO₂, SF₅ oder eine Alkyl-, Alkoxy-, Alkylcarbonyl- oder Alkoxycarbonylgruppe mit 1 bis 4 C-Atomen bedeutet, worin ein oder mehrere H-Atome mit F oder Cl substituiert sein können,
r 0, 1, 2, 3 oder 4 bedeutet,
R¹ jeweils unabhängig OH, O-B, OCH₂B bedeutet oder eine der unten angegebenen Bedeutungen von R besitzt,
k 0, 1, 2, 3 oder 4 bedeutet,
A und B unabhängig voneinander eine aromatische oder alicyclische Gruppe mit 4 bis 12 C-Atomen bedeuten,
Z jeweils unabhängig -O-, -S-, -CO-, -COO-, -OCO-, -CO-NR⁰-, -NR⁰-CO-, -CH₂CH₂-, -CF₂CF₂-, -CH₂CF₂-, -OCH₂-, -CH₂O-, -SCH₂-, -CH₂S-, -CF₂O-, -OCF₂-, -CH=CH-, -CF=CF-, -CH=CF-, -(CH₂)₄-, -CH=CH-COO-, -OCO-CH=CH-, -OOC-CHR⁰⁰-OOC-, -COO-CHR⁰⁰-COO-, -C≡C- oder eine Einfachbindung bedeutet,
R⁰⁰ geradkettiges oder verzweigtes Alkyl oder Alkoxy mit 1 bis 8 C-Atomen oder Phenyl bedeutet, welches gegebenenfalls ein- oder mehrfach durch L substituiert ist,
m 0, 1 oder 2 bedeutet,
R Halogen, NO₂, CN, SCN, SF₅, geradkettiges, verzweigtes oder cyclisches Alkyl mit 1 bis 25 C-Atomen bedeutet, worin eine oder mehrere CH₂-Gruppen auch so durch -O-, -S-, -CO-, -NR⁰-, -CH=CH-, -C≡C- ersetzt sein können, dass O- und/oder S-Atome nicht direkt miteinander verknüpft sind, und worin ein oder mehrere H-Atome auch durch F oder Cl oder Phenyl, das gegebenenfalls ein- oder mehrfach durch L substituiert ist, ersetzt sein können, oder P-(Sp-X)ₙ- bedeutet,
P eine polymerisierbare Gruppe ausgewählt aus CH₂=CW¹-COO-, 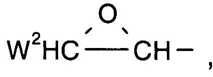 CH₂=CW²-O-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, OCN- und W⁴W⁵W⁶Si-bedeutet, wobei W¹ H, Cl, CN, Phenyl oder Alkyl mit 1 bis 5 C-Atomen, insbesondere H, Cl oder CH₃, bedeutet, W² und W³ unabhängig voneinander H oder Alkyl mit 1 bis 5 C-Atomen, insbesondere Methyl, Ethyl oder n-Propyl, bedeuten, W⁴, W⁵ und W⁶ unabhängig voneinander Cl, Oxaalkyl oder Oxacarbonylalkyl mit 1 bis 5 C-Atomen bedeuten, Phe 1,4-Phenylen bedeutet und k₁ und k₂ unabhängig voneinander 0 oder 1 bedeuten,
Sp eine Spacergruppe mit 1 bis 20 C-Atomen bedeutet,
X eine der Bedeutungen von Z besitzt und
n 0 oder 1 bedeutet,
mit der Maßgabe, dass m 1 oder 2 bedeutet und/oder mindestens eines von R und R¹ P-(Sp-X)ₙ- bedeutet,

2. Mischung nach Anspruch 1, worin M NR⁰, O, S, CH₂, (CH₂)₂, NR⁰-CH₂, O-CH₂ oder S-CH₂ bedeutet.

3. Mischung nach Anspruch 1 oder 2, worin m 1 oder 2 bedeutet.

4. Mischung nach einem beliebigen der Ansprüche 1 bis 3, worin mindestens eines von R und R¹ P-(Sp-X)ₙ- bedeutet.

5. Mischung nach einem beliebigen der Ansprüche 1 bis 4, worin
A jeweils unabhängig 1,4-Phenylen, bei dem zusätzlich eine oder mehrere CH-Gruppen durch N ersetzt sein können, 1,4-Cyclohexylen, bei dem zusätzlich eine oder zwei nicht benachbarte CH₂-Gruppen durch O und/oder S ersetzt sein können, 1,3-Dioxolan-4,5-diyl, 1,4-Cyclohexenylen, Piperidin-1,4-diyl, Pyrrolidin-2,4-diyl, 1,4-Bicyclo-(2,2,2)-octylen, Naphthalin-2,6-diyl, Decahydro-naphthalin-2,6-diyl, 1,2,3,4-Tetrahydronaphthalin-2,6-diyl oder Indan-2,5-diyl bedeutet, wobei alle diese Gruppen unsubstituiert oder ein- oder mehrfach mit L wie in Formel I definiert substituiert sein können.

6. Mischung nach einem beliebigen der Ansprüche 1 bis 5, worin die Gruppe 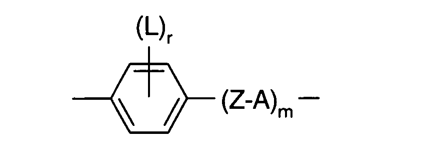 in Formel I ausgewählt ist aus den folgenden Formeln 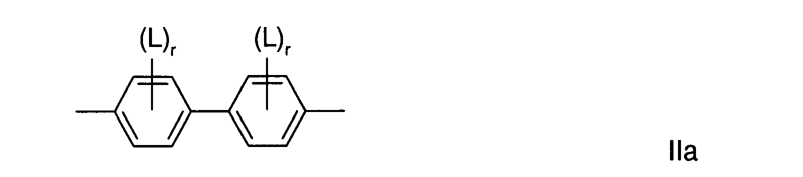 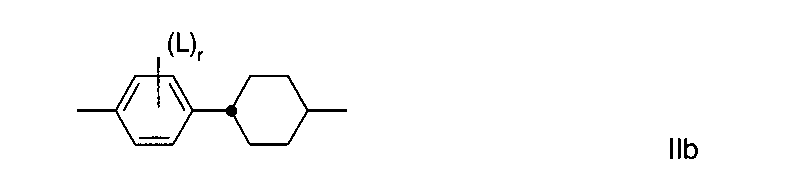 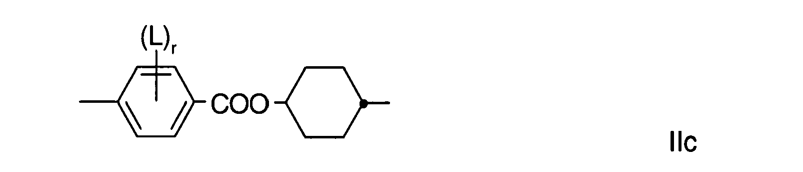 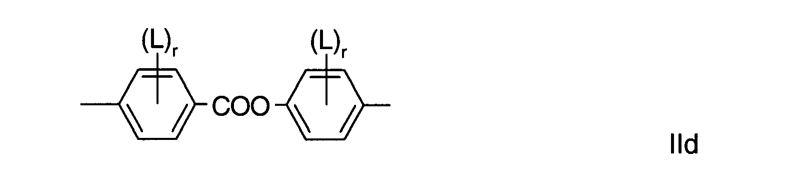 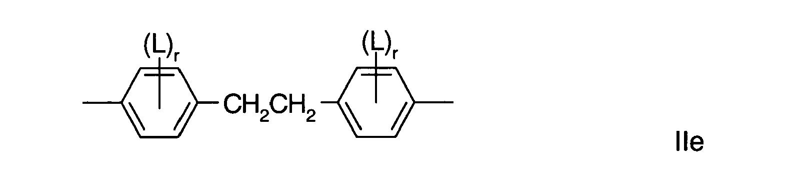 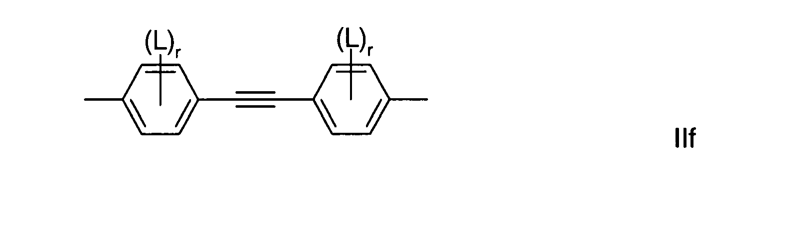 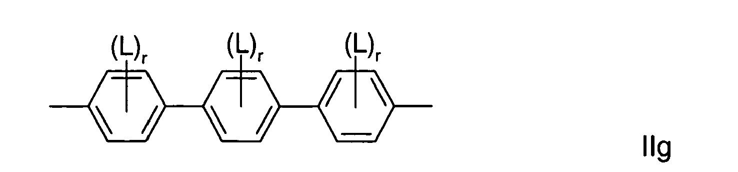 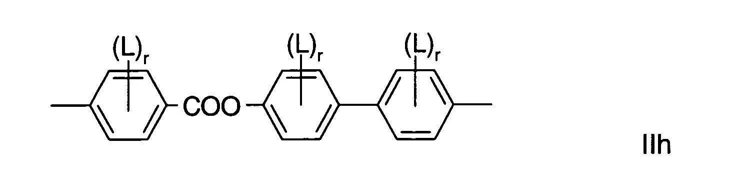 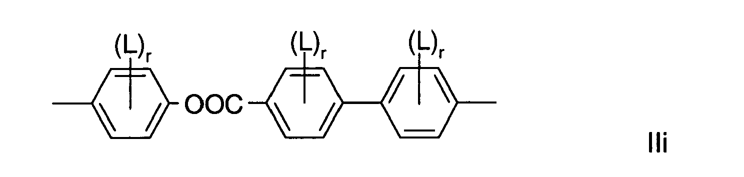 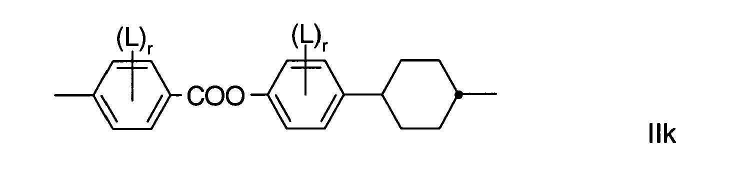 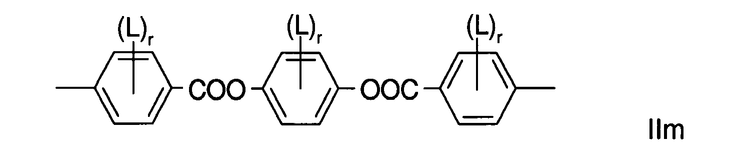 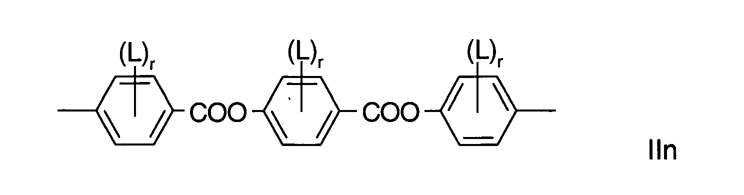 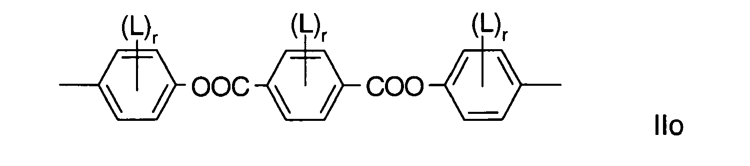 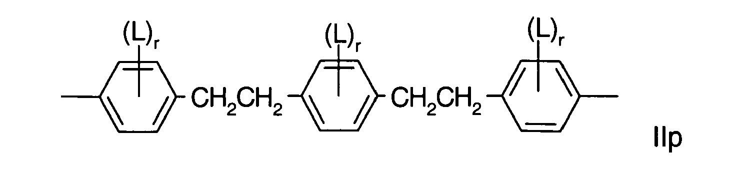 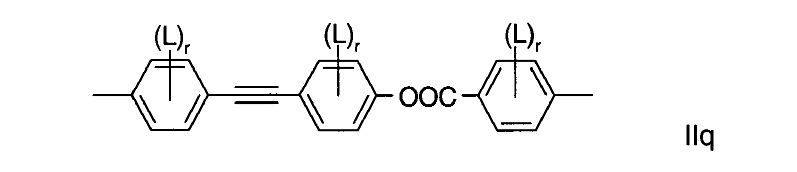 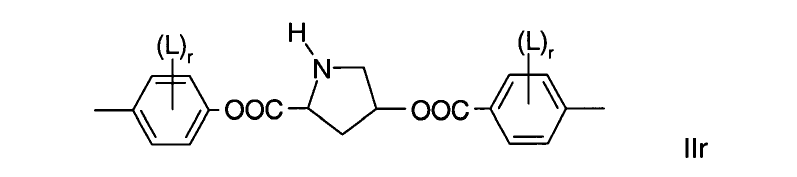 worin L die Bedeutung der Formel I besitzt und r 0, 1 oder 2 bedeutet.

7. Mischung nach einem beliebigen der Ansprüche 1 bis 6, worin die Verbindung der Formel I ausgewählt ist aus den folgenden Unterformeln 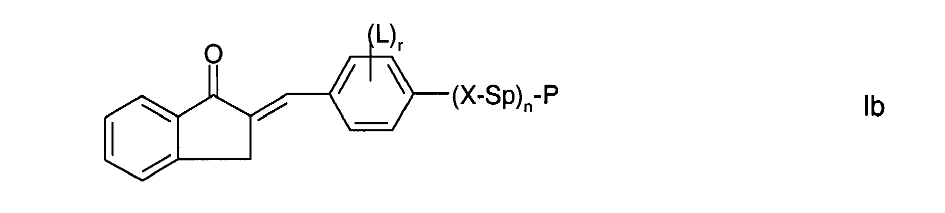 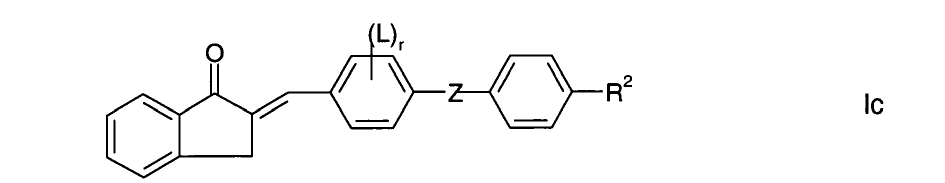 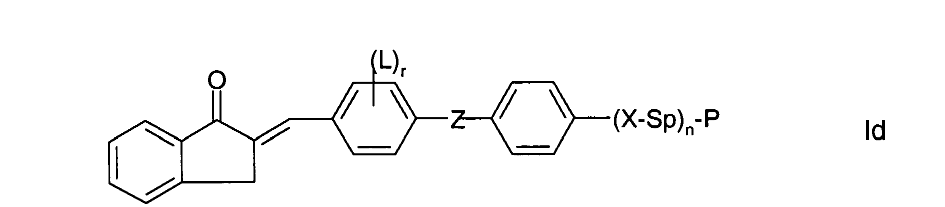 worin Z, P, Sp, X, L, n und r wie in Formel I definiert sind und
R² is H, Halogen, NO₂, CN, SCN, SF₅, geradkettiges, verzweigtes oder cyclisches Alkyl mit 1 bis 25 C-Atomen bedeutet, worin eine oder mehrere CH₂-Gruppen auch so durch -O-, -S-, -CO-, -NR⁰-, -CH=CH-, -C≡C- ersetzt sein können, dass O-und/oder S-Atome nicht direkt miteinander verknüpft sind, und worin ein oder mehrere H-Atome auch durch F oder Cl ersetzt sein können.

8. Mischung nach einem beliebigen der Ansprüche 1 bis 7, worin P eine Acrylat-, Methacrylat-, Vinyloxy-, Epoxy-, Styrol- oder Propenylethergruppe bedeutet.

9. Mischung nach einem beliebigen der Ansprüche 1 bis 8, worin R und/oder R¹ eine Alkyl- oder Alkoxygruppe mit 1 bis 12 C-Atomen bedeutet.

10. Mischung nach einem beliebigen der Ansprüche 1 bis 9, enthaltend mindestens eine polymerisierbare Verbindung ausgewählt aus den folgenden Formeln 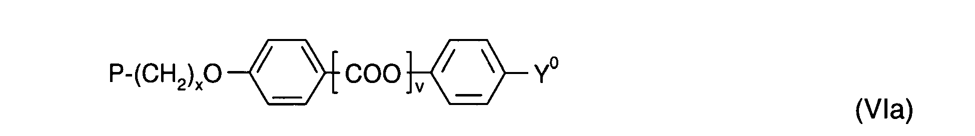 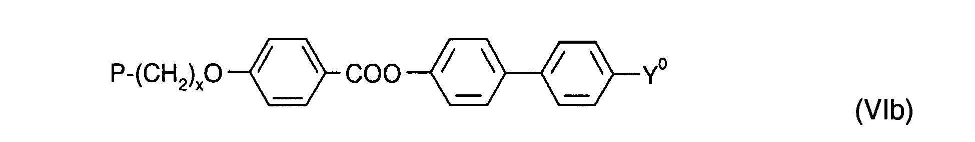 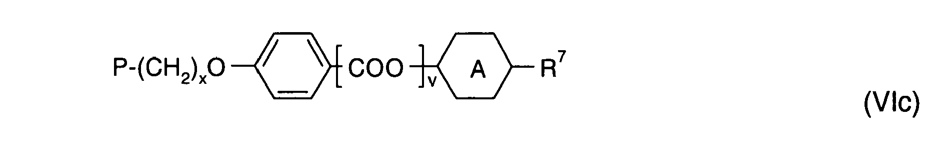 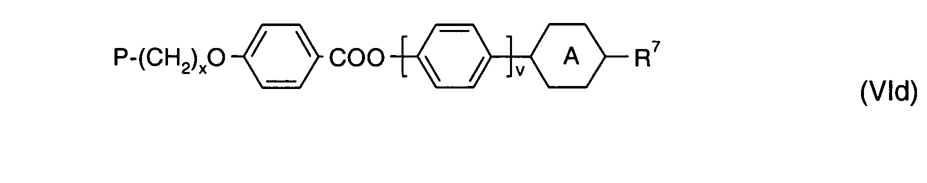 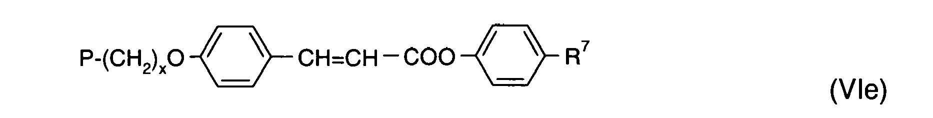 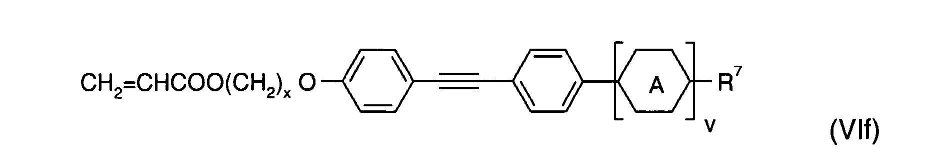 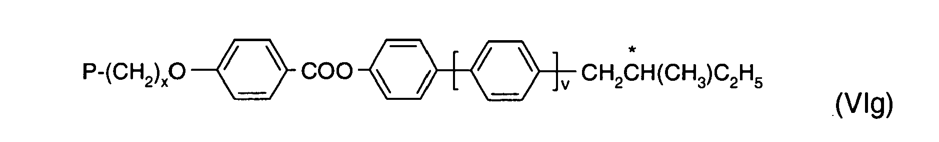 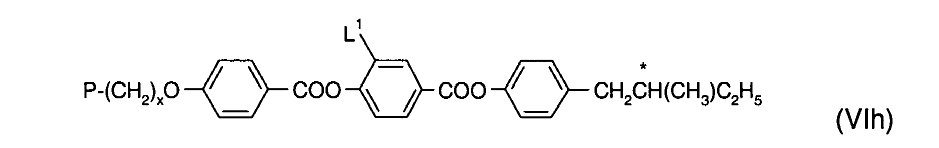 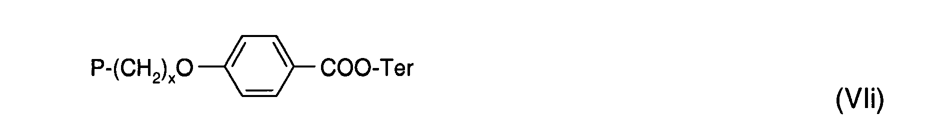 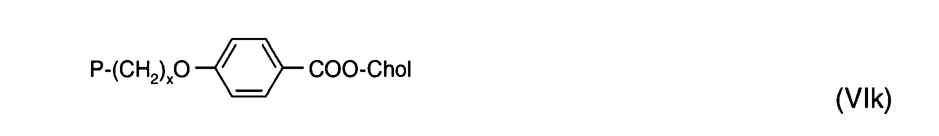 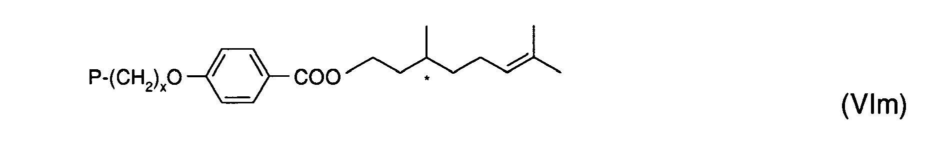 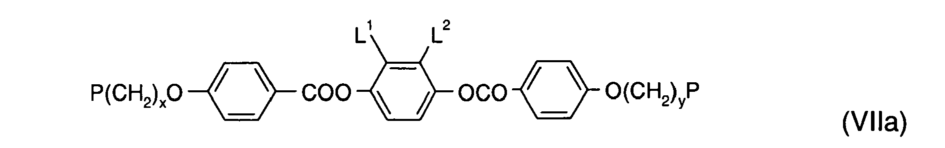 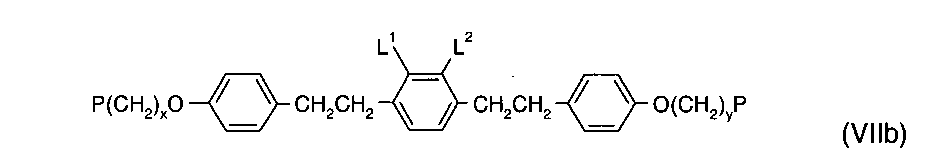 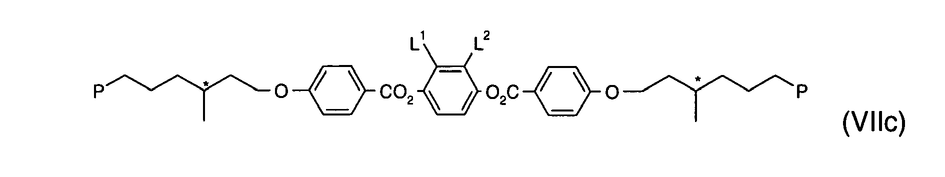 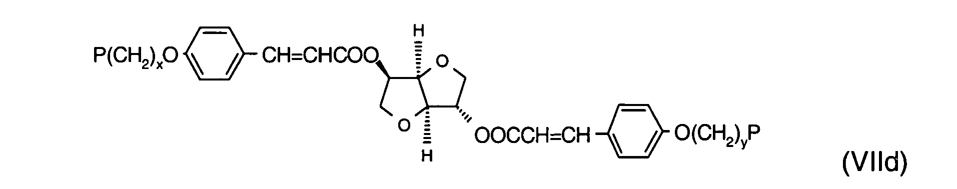 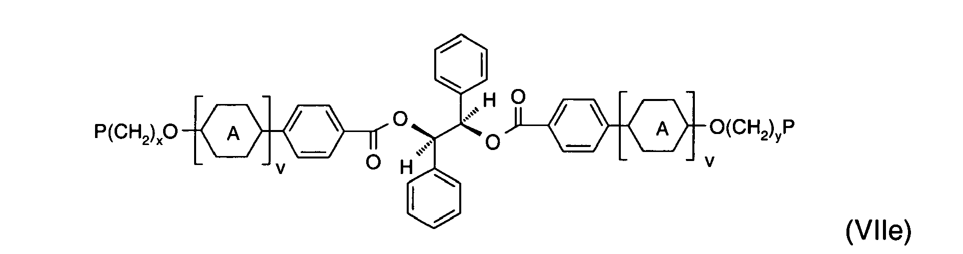 worin
P eine der Bedeutungen des Anspruchs 1 besitzt,
x eine ganze Zahl von 1 bis 12 bedeutet,
y eine ganze Zahl von 1 bis 12 bedeutet, die gleich an oder verschieden von x ist ,
A 1,4-Phenylen oder 1,4-Cyclohexylen bedeutet,
v 0 oder 1 bedeutet,
Y⁰ CN, NO₂, Halogen, OCH₃, OCN, SCN, COR⁸, COOR⁸ oder eine mono-, oligo- oder polyfluorierte Alkyl- oder Alkoxygruppe mit 1 bis 4 C-Atomen bedeutet,
R⁸ gegebenenfalls fluoriertes Alkyl mit 1 bis 4 C-Atomen bedeutet,
R⁷ eine Alkylgruppe mit 1 bis 15 C-Atomen oder eine Alkoxygruppe mit 2 bis 15 C-Atomen bedeutet,
Ter einen Terpenoidrest bedeutet,
Chol eine Cholesterylgruppe bedeutet und
L¹ und L² jeweils unabhängig H, F, Cl, CN, OH, NO₂ oder eine gegebenenfalls halogenierte Alkyl-, Alkoxy- oder Carbonylgruppe mit 1 bis 7 C-Atomen bedeuten.

11. Lineares oder vernetztes anisotropes Polymer erhältlich durch Polymerisieren einer Mischung nach einem beliebigen der Ansprüche 1 bis 10.

12. Verbindung der Formel I 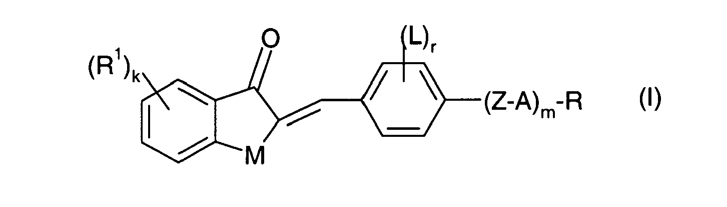 worin M, R, R¹, A, Z, L, k, m und r wie in einem beliebigen der Ansprüche 1 bis 9 definiert sind und worin mindestens eines von R und R¹ P-(Sp-X)ₙ- bedeutet.

13. Verwendung photoisomerisierbarer Verbindungen, Mischungen und Polymere nach den Ansprüchen 1 bis 12 in optischen und elektrooptischen Vorrichtungen wie Flüssigkristallanzeigen oder Projektionssystemen, gemusterten Folien und optischen Elementen wie Polarisatoren, Verzögerungsfolien, Kompensatoren, Farbfiltern, holographischen Elementen oder Polarisationsstrahlteilern, als Sensibilisierer, zum Photoschalten, in anisotropen Membranen für die Gas- oder Flüssigkeitspermeation, Klebstoffen, Kunstharzen mit anisotropen mechanischen Eigenschaften, kosmetischen oder pharmazeutischen Zusammensetzungen, UV-Absorbern und Sonnenschutzmitteln, Diagnostika oder Flüssigkristallpigmenten, für dekorative und Sicherheitsanwendungen, in der nichtlinearen Optik, optischen Datenspeicherung oder als Dotierstoffe.

14. Flüssigkristallanzeige enthaltend eine Verbindung, eine Mischung oder ein Polymer nach einem beliebigen der Ansprüche 1 bis 12.

15. Polymerfolie erhältlich durch schichtförmiges Auftragen einer polymerisierbaren Mischung nach einen beliebigen der Ansprüche 1 bis 10 auf ein Substrat, Orientieren der Mischung in eine uniforme Ausrichtung und Polymerisieren der Mischung, wobei mindestens ein Teil der Mischung vor oder während der Polymerisation aktinischer Strahlung einer Wellenlänge ausgesetzt wird, bei der die Verbindung der Formel I Photoisomerisierung zeigt.

16. Polymerfolie nach Anspruch 15, die ein Muster von mindestens zwei Bereichen mit unterschiedlicher Ausrichtung aufweist.

17. Polymerfolie nach Anspruch 15, die ein Muster von mindestens zwei Bereichen mit unterschiedlicher Doppelbrechung aufweist.

18. Polymerfolie nach Anspruch 15, die ein Muster von mindestens einem Bereich mit ausgerichtetem Zustand und mindestens einem Bereich mit teilweise oder gänzlich nicht ausgerichtetem Zustand aufweist.

19. Polymerfolie nach einem beliebigen der Ansprüche 15 bis 18, **dadurch gekennzeichnet, dass** es sich um eine nematische Folie handelt.

20. Polymerfolie nach Anspruch 15, **dadurch gekennzeichnet, dass** es sich um eine gemusterte cholesterische Folie mit planarer Orientierung und Bereichen unterschiedlicher Reflexionswellenlänge handelt.

21. Polymerfolie nach Anspruch 15, **dadurch gekennzeichnet, dass** es sich um eine breitbandig reflektierende cholesterische Folie handelt.

22. Polymerfolie nach Anspruch 15, **dadurch gekennzeichnet, dass** es sich um eine cholesterische Folie handelt, bei der die Ganghöhe in horizontaler oder vertikaler Richtung variiert und die aus einer achiralen isomerisierbaren Verbindung der Formel I zusammen mit einer cholesterischen Flüssigkristallmischung hergestellt wird.

23. Farbfilter, Sicherheitsmarkierung, dekorative Beschichtung oder breitbandig reflektierender Polarisator enthaltend eine Polymerfolie nach einem beliebigen der Ansprüche 15 bis 22.

## Revendications

1. Mélange cristallin liquide polymérisable comprenant au moins un composé de la formule I, et comprenant au moins un composé polymérisable, qui peut être un composé de la formule I ou un composé polymérisable additionnel 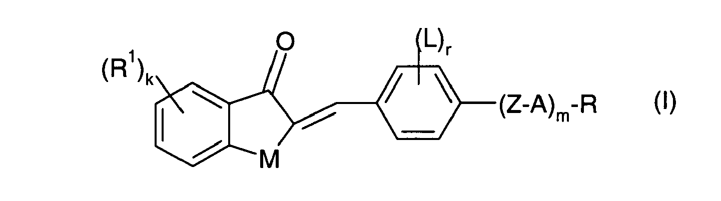 dans laquelle
M est (CH₂)ᵢ, dans lequel un groupe CH₂ peut également être remplacé par NR⁰, O ou S,
i est 1, 2, 3 ou 4,
R⁰ est H ou alkyle avec 1 à 4 atomes de C,
L est halogène, CN, SCN, NO₂, SF₅ ou un groupe alkyle, alcoxy, alkylcarbonyle ou alkoxycarbonyle avec 1 à 4 atomes de C, dans lequel un ou plusieurs atomes de H peuvent être substitués avec F ou Cl.
r est 0, 1, 2, 3 ou 4,
R¹ est dans chaque cas indépendamment OH, O-B, OCH₂B ou a une des significations de R données ci-après,
k est 0, 1, 2, 3 ou 4,
A et B sont indépendamment l'un de l'autre un groupe aromatique ou alicyclique avec 4 à 12 atomes de C,
Z est dans chaque cas indépendamment -O-, -S-, -CO-, -COO-, -OCO-, -CO-NR⁰-, -NR⁰-CO-, -CH₂CH₂-, -CF₂CF₂-, -CH₂CF₂-, -OCH₂-, -CH₂O-, -SCH₂-, -CH₂S-, -CF₂O-, -OCF₂-, -CH=CH-, -CF=CF-, -CH=CF-, -(CH₂)₄-, -CH=CH-COO-, -OCO-CH=CH-, -OOC-CHR⁰⁰-OOC-, -COO-CHR⁰⁰-COO-, -C≡C- ou une liaison simple,
R⁰⁰ est alkyle ou alcoxy en chaîne droite ou ramifié avec 1 à 8 atomes de C ou phényle qui est optionnellement mono- ou polysubstitué par L,
m est 0, 1 ou 2,
R est halogène, NO₂, CN, SCN, SF₅, alkyle en chaîne droite, ramifié ou cyclique avec 1 à 25 atomes de C dans lequel un ou plusieurs groupes CH₂ peuvent également être remplacés par -O-, -S-, -CO-, -NR⁰-, -CH=CH-, -C≡C- de telle sorte que des atomes de O et/ou S ne soient pas liés directement les uns aux autres, et dans lequel un ou plusieurs atomes de H peuvent également être remplacés par F ou Cl ou phényle qui est optionnellement mono- ou polysubstitué par L, ou représente P-(Sp-X)ₙ-,
P est un groupe polymérisable choisi parmi CH₂=CW¹-COO-, 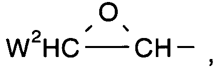 CH₂=CW²-O-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, OCN- et W⁴W⁵W⁶Si-, avec W¹ étant H, Cl, CN, phényle ou alkyle avec 1 à 5 atomes de C, en particulier H, Cl ou CH₃, W² et W³ étant indépendamment l'un de l'autre H ou alkyle avec 1 à 5 atomes de C, en particulier méthyle, éthyle ou n-propyle, W⁴, W⁵ et W⁶ étant indépendamment les uns des autres Cl, oxaalkyle ou oxacarbonylalkyle avec 1 à 5 atomes de C, Phe étant 1,4-phénylène et k₁ et k₂ étant indépendamment l'un de l'autre 0 ou 1,
Sp est a groupe espaceur avec 1 à 20 atomes de C,
X a une des significations de Z, et
n est 0 ou 1,
avec la réserve que m est 1 ou 2 et/ou au moins un de R et R¹ représente P-(Sp-X)ₙ-,

2. Mélange selon la revendication 1, dans lequel M est NR⁰, O, S, CH₂, (CH₂)₂, NR⁰-CH₂, O-CH₂ ou S-CH₂.

3. Mélange selon la revendication 1 ou 2, dans lequel m est 1 ou 2.

4. Mélange selon l'une quelconque des revendications 1 à 3, dans lequel au moins un de R et R¹ représente P-(Sp-X)ₙ-.

5. Mélange selon l'une quelconque des revendications 1 à 4, dans lequel
A est dans chaque cas indépendamment 1,4-phénylène dans lequel, en addition, un ou plusieurs groupes CH peuvent être remplacés par N, 1,4-cyclohexylène dans lequel, en addition, un ou deux groupes CH₂ non-adjacents peuvent être remplacés par O et/ou S, 1,3-dioxolane-4,5-diyle, 1,4-cyclohexénylène, pipéridine-1,4-diyle, pyrrolidine-2,4-diyle, 1,4-bicyclo-(2,2,2)-octylène, naphtalène-2,6-diyle, décahydronaphtalène-2,6-diyle, 1,2,3,4-tétrahydronaphtalène-2,6-diyle ou indane-2,5-diyle, étant possible pour tous ces groupes qu'ils soient non substitués, mono- ou polysubstitués avec L comme défini dans la formule I.

6. Mélange selon l'une quelconque des revendications 1 à 5, dans lequel le groupe 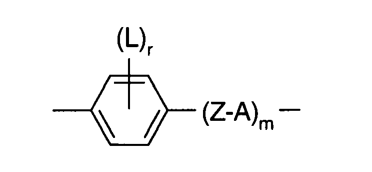 dans la formule I est choisi parmi les formules suivantes 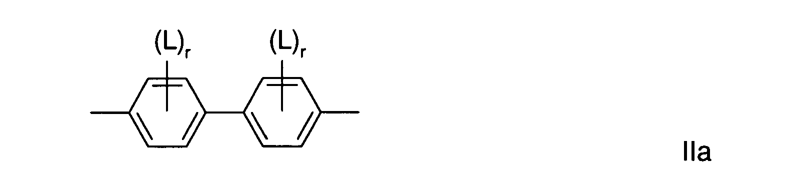 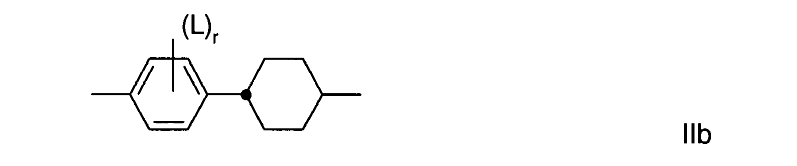 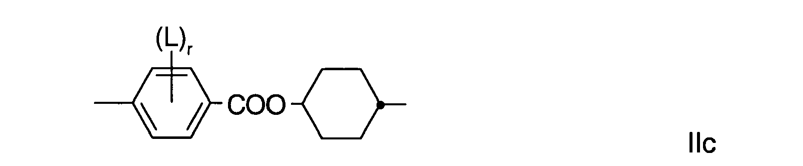 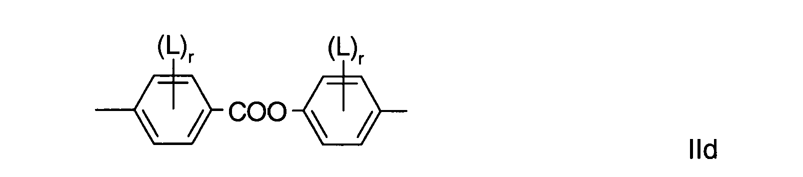 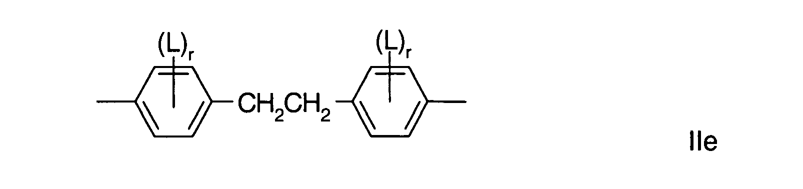 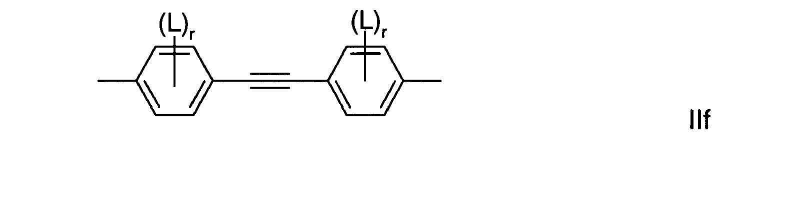 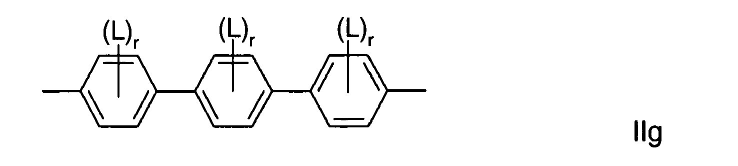 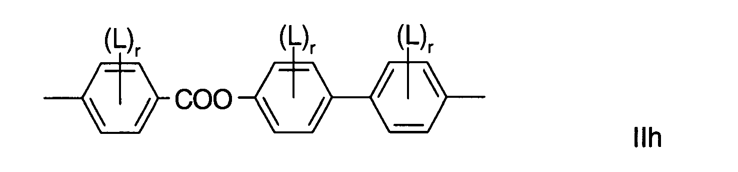 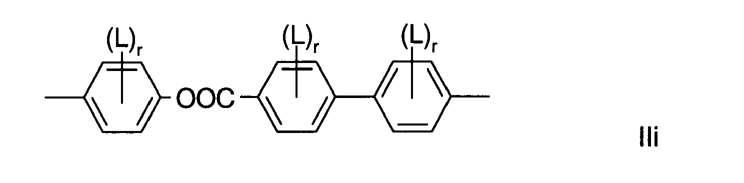 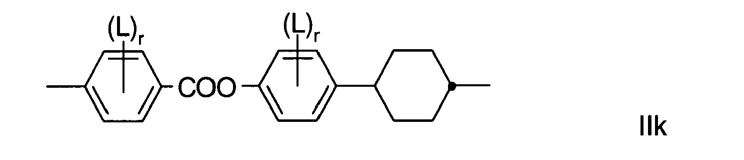 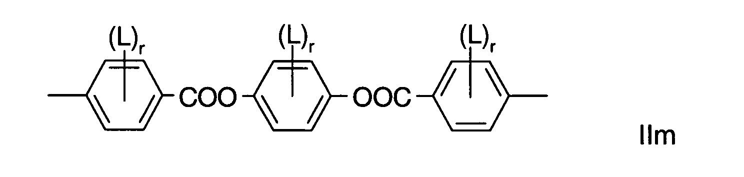 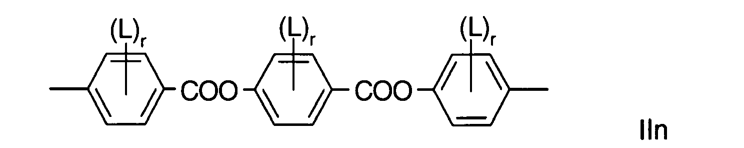 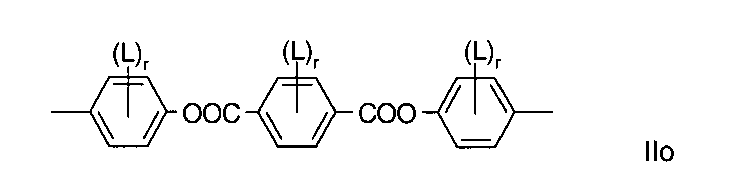 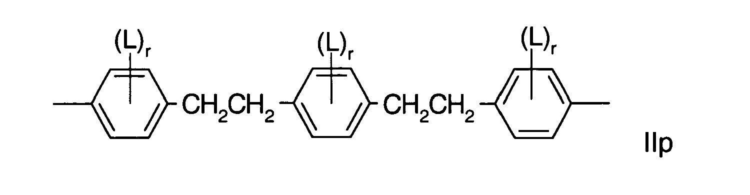 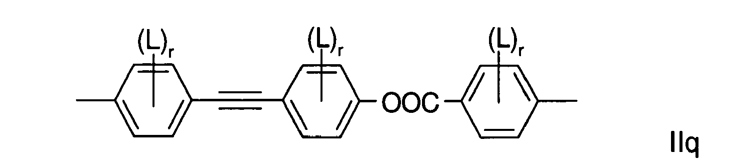 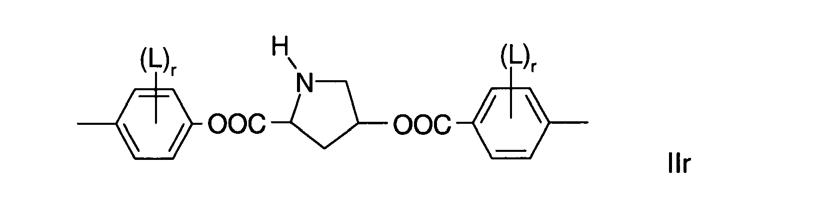 dans lesquelles L a la signification de la formule I et r est 0, 1 ou 2.

7. Mélange selon l'une quelconque des revendications 1 à 6, dans lequel le composé de la formule I est choisi parmi les sous-formules suivantes 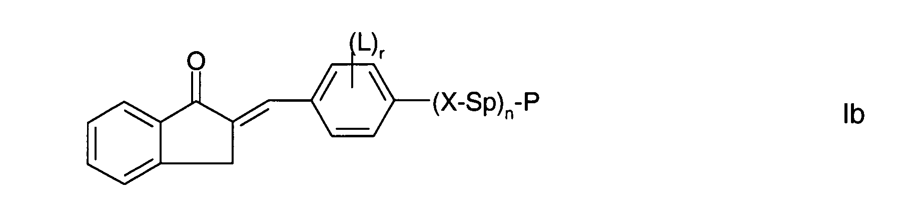 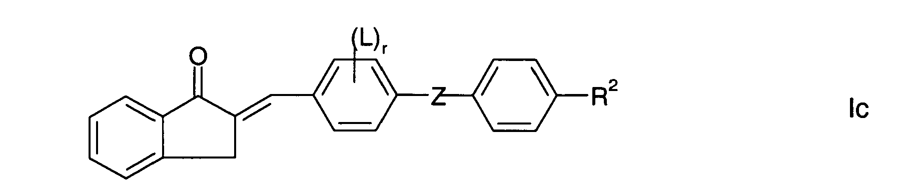 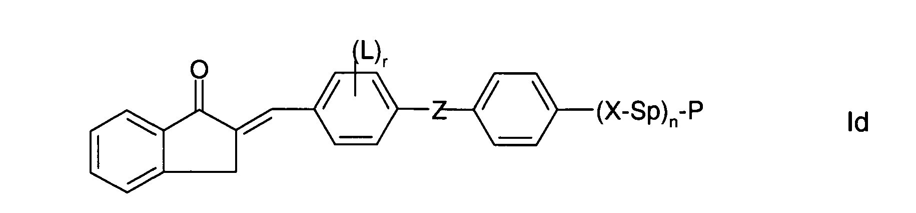 dans lesquelles Z, P, Sp, X, L, n et r sont comme définis dans la formule I et
R² est H, halogène, NO₂, CN, SCN, SF₅, alkyle en chaîne droite, ramifié ou cyclique avec 1 à 25 atomes de C dans lequel un ou plusieurs groupes CH₂ peuvent également être remplacés par -O-, -S-, -CO-, -NR⁰-, -CH=CH-, -C≡C- de telle sorte que des atomes de O et/ou S ne soient pas liés directement les uns aux autres, et dans lequel un ou plusieurs atomes de H peuvent également être remplacés par F ou Cl.

8. Mélange selon l'une quelconque des revendications 1 à 7, dans lequel P est un groupe acrylate, méthacrylate, vinyloxy, époxy, styrène ou propényléther.

9. Mélange selon l'une quelconque des revendications 1 à 8, dans lequel R et/ou R¹ est un groupe alkyle ou alcoxy avec 1 à 12 atomes de C.

10. Mélange selon l'une quelconque des revendications 1 à 9, comprenant au moins un composé polymérisable choisi parmi les formules suivantes 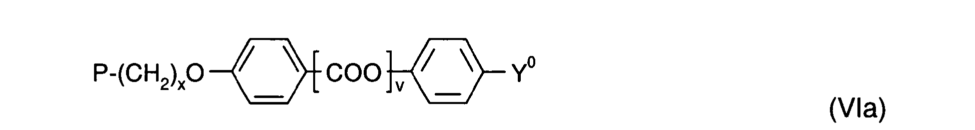 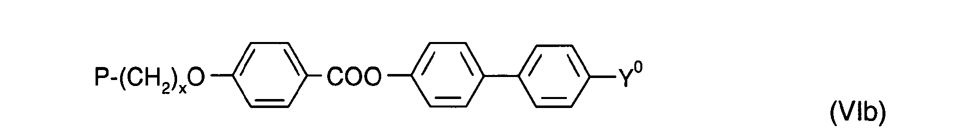 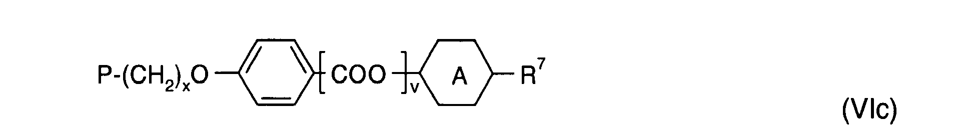 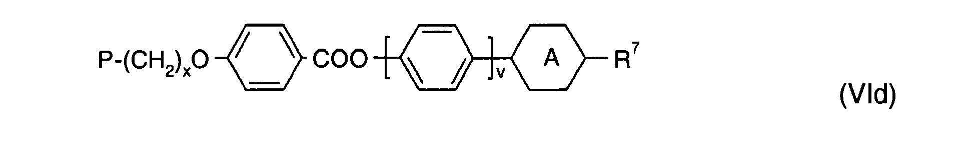 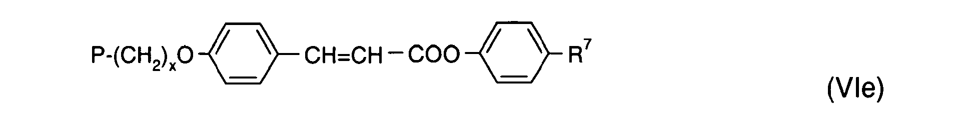 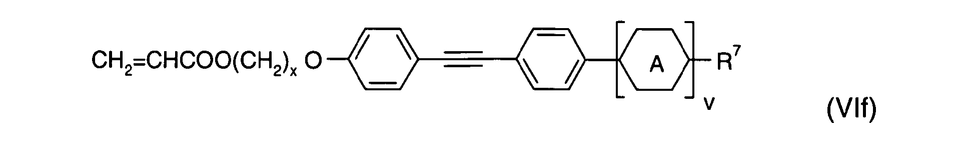 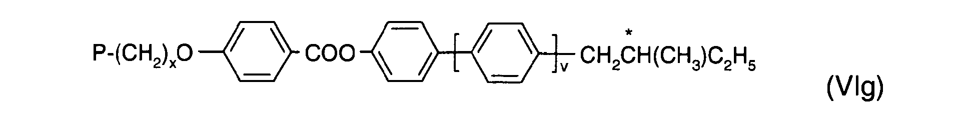 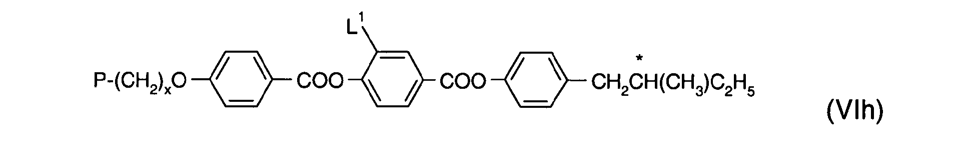 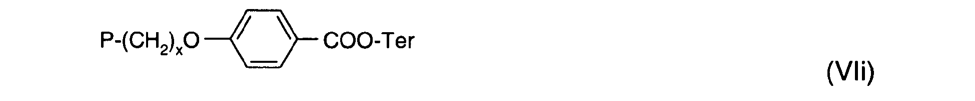 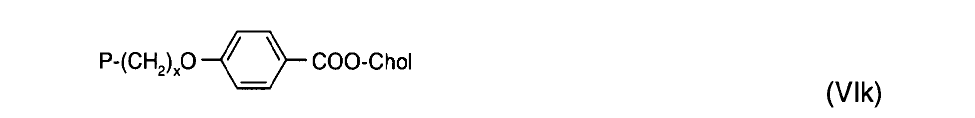 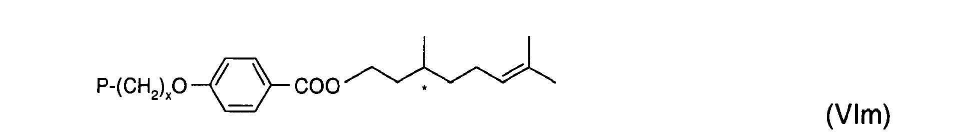 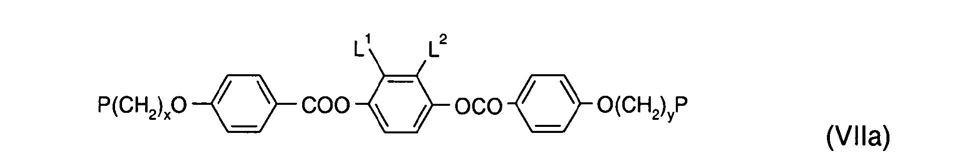 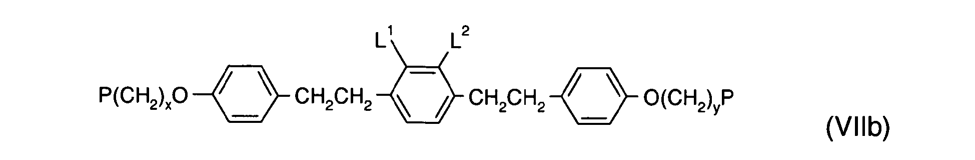 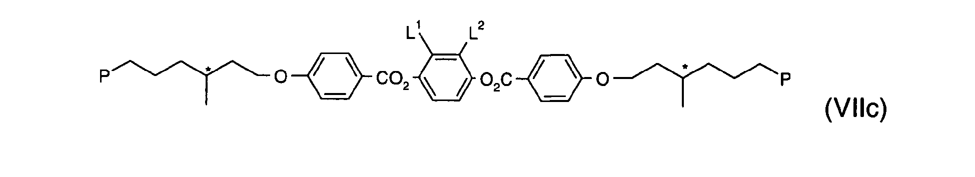 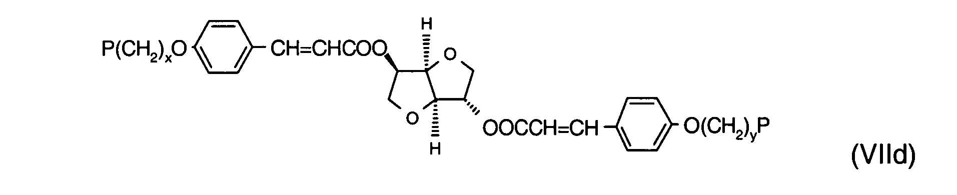 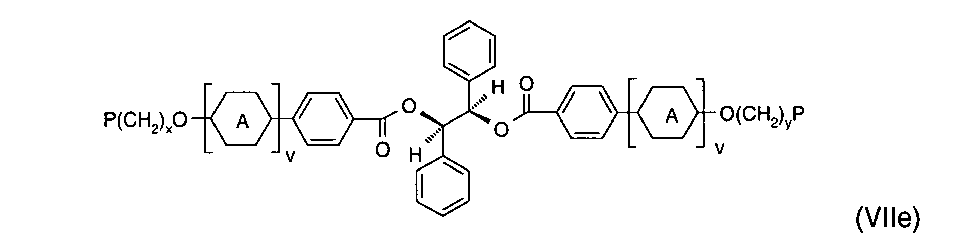 dans lesquelles
P a une des significations de la revendication 1,
x est un entier de 1 à 12 ,
y est un entier de 1 à 12 qui est le même ou différent de x,
A est 1,4-phénylène ou 1,4-cyclohexylène,
v est 0 ou 1,
Y⁰ est CN, NO₂, halogène, OCH₃, OCN, SCN, COR⁸, COOR⁸ ou un groupe alkyle ou alcoxy mono-, oligo- ou polyfluoré avec 1 à 4 atomes de C,
R⁸ est optionnellement alkyle fluoré avec 1 à 4 atomes de C
R⁷ est un groupe alkyle avec 1 à 15 atomes de C ou un groupe alcoxy avec 2 à 15 atomes de C,
Ter est un radical terpénoïde,
Chol est un groupe cholestéryle, et
L¹ et L² sont chacun indépendamment H, F, Cl, CN, OH, NO₂ ou un groupe alkyle, alcoxy ou carbonyle optionnellement halogéné avec 1 à 7 atomes de C.

11. Polymère anisotrope linéaire ou réticulé pouvant être obtenu en polymérisant un mélange selon l'une quelconque des revendications 1 à 10.

12. Composé de la formule I 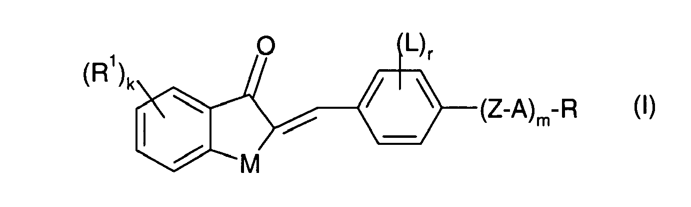 dans laquelle M, R, R¹, A, Z, L, k, m et r sont comme définis dans l'une quelconque des revendications 1 à 9, et dans lequel au moins un de R et R¹ représente P-(Sp-X)ₙ-.

13. Utilisation de composés, mélanges et polymères photo-isomérisables selon les revendications 1 à 12 dans des dispositifs optiques et électrooptiques tels que des affichages ou systèmes de projection à cristaux liquides, films conformés et éléments optiques tels que des polariseurs, films de retardement, compensateurs, filtres couleur, éléments holographiques ou séparateurs de faisceau de polarisation, en tant que détecteurs, pour la photocommutation, dans des membranes anisotropes pour la perméation de gaz ou de fluides, adhésifs, résines synthétiques avec des propriétés mécaniques anisotropes, compositions cosmétiques ou pharmaceutiques, absorbeurs UV et écrans solaires, pigments de diagnostique ou de cristal liquide, pour des applications décoratives et de sécurité, dans des optiques non linéaires, des mémoires d'information optiques ou en tant que dopants.

14. Affichage à cristaux liquides comprenant un composé, mélange ou polymère selon l'une quelconque des revendications 1 à 12.

15. Film polymère pouvant être obtenu en revêtant un mélange polymérisable selon l'une quelconque des revendications 1 à 10 sur un substrat, en alignant le mélange selon une orientation uniforme, et en polymérisant le mélange, dans lequel au moins une partie du mélange avant ou pendant la polymérisation est exposée à un rayonnement actinique d'une longueur d'onde où le composé de la formule I présente une photo-isomérisation.

16. Film polymère selon la revendication 15 présentant un motif d'au moins deux régions ayant des orientations différentes.

17. Film polymère selon la revendication 15 présentant un motif d'au moins deux régions ayant des biréfringences différentes.

18. Film polymère selon la revendication 15 présentant un motif d'au moins une région avec un état orienté et d'au moins une région avec un état partiellement ou totalement orienté.

19. Film polymère selon l'une quelconque des revendications 15 à 18, **caractérisé en ce qu'**il est un film nématique.

20. Film polymère selon la revendication 15, **caractérisé en ce qu'**il est un film cholestérique conformé ayant un alignement plan et des régions de longueurs d'onde de réflexion différentes.

21. Film polymère selon la revendication 15, **caractérisé en ce qu'**il est un film cholestérique réflectif large bande.

22. Film polymère selon la revendication 15, **caractérisé en ce qu'**il est un film cholestérique dans lequel le pas varie dans la direction horizontale ou verticale, préparé à partir d'un composé isomérisable achiral de la formule I en association avec un mélange de cristal liquide cholestérique.

23. Filtre couleur, marquage de sécurité, revêtement décoratif ou polariseur réflectif large bande comprenant un film polymère selon l'une quelconque des revendications 15 à 22.
